# EUROPEAN PATENT SPECIFICATION

(11) **EP 4 158 638 B1**
(45) Date of publication and mention of the grant of the patent: **28.10.2026**
(21) Application number: 21813018.5
(22) Date of filing: 27.05.2021
(51) Int. Cl.: G16B 40/20, G16B 20/20, G16B 20/40, G06N 5/04, G16H 50/30, G16H 50/70

(54) **MACHINE LEARNING PLATFORM FOR GENERATING RISK MODELS**
MASCHINENLERNPLATTFORM ZUR ERZEUGUNG VON RISIKOMODELLEN
PLATE-FORME D'APPRENTISSAGE AUTOMATIQUE POUR GÉNÉRATION DE MODÈLES DE RISQUE

(30) Priority: 27.05.2020 US 202063030876 P
(43) Date of publication of application: 05.04.2023
(73) Proprietor: 23andMe Genomics LLC, Los Altos, CA 94022 (US)
(72) Inventor: POLCARI, Michael, Sunnyvale, California 94086 (US); ZHAN, Jianan, Sunnyvale, California 94086 (US); GANESAN, Manoj, Sunnyvale, California 94086 (US); MARSHALL, Austin William, Sunnyvale, California 94086 (US); ASHENHURST, James Rowan, Sunnyvale, California 94086 (US); KONDO, Derrick Poo-Ray, Sunnyvale, California 94086 (US); AMIRI, Shiva, Sunnyvale, California 94086 (US); SINHA, Subarnarekha, Sunnyvale, California 94086 (US); SURESH, Sanjeev, Sunnyvale, California 94086 (US); MACPHERSON, John Michael, Sunnyvale, California 94086 (US); KOELSCH, Bertram Lorenz, Sunnyvale, California 94086 (US); BLAKKAN, Cordell T., Sunnyvale, California 94086 (US); HAMILTON, Shannon M., Sunnyvale, California 94086 (US)
(74) Representative: Carpmaels & Ransford LLP
(86) International application number: PCT/US2021/034634
(87) International publication number: WO 2021/243094

(56) References cited:
- WO-A1-2019/236392
- US-A1- 2017 337 483
- US-A1- 2018 107 785
- US-A1- 2019 164 313
- US-A1- 2019 264 285
- US-A1- 2020 118 647
- US-A1- 2020 118 647
- HAMID BEHRAVAN ET AL: "Machine learning identifies interacting genetic variants contributing to breast cancer risk: A case study in Finnish cases and controls", SCIENTIFIC REPORTS, vol. 8, no. 1, 3 September 2018 (2018-09-03), XP055719162, DOI: 10.1038/s41598-018-31573-5

## Description

### BACKGROUND

Polygenic Risk Scores (PRS or PGS) are probabilities for an individual to have a specific phenotype. PRSes for a particular user may be determined by leveraging large databases of genomic and phenotypic data for research consenting customers. These data can be leveraged to identify meaningful associations of particular genetic loci with a particular phenotype, and to model the combined effect of these genetic loci in the overall probability for an individual to have the specific phenotype.

US 2020/118647 A1 describes a system and method that predicts a phenotypic trait for an individual. The system identifies a subset of SNP loci with predictive ability of the phenotypic trait. The system calculates a PRS for the individual based on the individual's genetic dataset at the identified subset of SNP loci. The system compares the PRS to a threshold PRS. The threshold PRS is determined by calculating, for each training individual of a plurality of training individuals including some reported to have and some reported to not have the phenotypic trait, a PRS, sweeping through a domain of PRS while calculating a true positive rate and a false positive rate, and then identifying an optimal threshold PRS as the threshold PRS. The system generates a prediction whether the individual has the phenotypic trait based on the comparison.

### SUMMARY

Disclosed herein are methods and systems of generating PRS models using an end to end PRS machine.. The invention is defined in the appended set of claims. A method for generating a polygenic risk score (PRS) model to predict phenotypes of a user is provided, the method including: receiving
user-selected parameters related to a PRS model including a phenotype of interest; selecting and obtaining genetic data for a plurality of individuals based on the user selected parameters, the genetic data including data pertaining to a presence or absence of the phenotype of interest in the plurality of individuals; determining a plurality of population-specific genetic datasets from the genetic data based on the plurality of individuals; analyzing the population-specific genetic datasets to determine one or more sets of SNPs that are statistically associated with the phenotype of interest for that population-specific genetic dataset, wherein each set of SNPs corresponds with one population-specific genetic dataset; applying SNP filtering criteria to the one or more sets of SNPs to generate a plurality of training SNP sets, wherein the SNP filtering criteria is based at least in part on the user-selected parameters; loading into a cache of a computer system genetic and phenotypic information for the plurality of individuals in each population-specific genetic dataset; training a plurality of models using machine-learning techniques based at least in part on the genetic and phenotypic information for the plurality of individuals in the cache, the plurality of training SNP sets, and the phenotype of interest; generating one or more performance metrics for each of the plurality of models for each of the population-specific genetic datasets; and determining a plurality of population-specific models based on the one or more performance metrics.

In some embodiments, the method further includes determining a combined set of SNPs that may be statistically associated with the phenotype of interest based at least in part on a meta-analysis of the one or more sets of SNPs. In some embodiments, the method further includes determining the combined set of SNPS based on an inverse weighting of the one or more sets of SNPs. In some embodiments, the method further includes determining the combined set of SNPS based on scores from other polygenic models for at least some of the plurality of individuals. In some embodiments, the method further includes determining the plurality of individuals by filtering a dataset of individuals based on the user-selected parameters. In some embodiments, the user-selected parameters may include one or more of: research consent status, missing SNP values, relatedness to other individuals in the dataset of individuals, minimum age, maximum age, sequencing platform, sex, and population classifier label. In some embodiments, the method further includes: receiving phenotype data for the plurality of individuals, and additionally analyzing the phenotype data of the plurality of individuals to determine the plurality of SNPs that may be statistically associated with a phenotype of interest. In some embodiments, the phenotype data may include one or more of: answers to survey questions, family history, medical records, biomarkers, and data from one or more wearable sensors. In some embodiments, the genetic data may include one or more of: directly genotyped data, imputed genetic data, next generation sequencing data, whole genome sequencing data, and functionally aggregated data. In some embodiments, the genetic data may include imputed data with greater than about 50,000,000 variants per individual, greater than about 75,000,000 variants per individual, or greater than about 100,000,000 variants per individual. In some embodiments, a database storing the genetic data may include genetic data for greater than 10,000,000 individuals. In some embodiments, the method further includes dividing one or more of the population-specific datasets into a training set, validation set, and test set, wherein analyzing one or more of the population-specific datasets is the genetic data of the plurality of individuals in the training sets. In some embodiments, dividing each of the population-specific datasets is based on the user-selected parameters. In some embodiments, the method further includes: determining that a number of individuals in a first population-specific dataset does not exceed a first threshold; and identifying the first population-specific dataset as a test set. In some embodiments, the method further includes: determining that a number of individuals in a second population-specific dataset does not exceed a second threshold; and dividing the second population-specific dataset into a training set and validation set.

In some embodiments, the generating one or more performance metrics is based on the genetic data and phenotype data of the plurality of individuals in the validation set. In some embodiments, the method further includes analyzing the population-specific models based on the genetic data and phenotype data of the plurality of individuals in the population-specific test set. In some embodiments, analyzing at least the genetic data may include running a genome wide association study (GWAS) on the genetic data and the phenotype of interest. In some embodiments, running the GWAS may include separating the plurality of individuals into case and control groups based on the user selected parameters. In some embodiments, the filtering criteria may include one or more of: allow listing, distance pruning, p-value threshold, and linkage disequilibrium pruning. In some embodiments, the cache of the computer system may include genetic and phenotypic information for at least about 1,000,000 individuals, at least about 500,000 individuals, or at least about 100,000 individuals. In some embodiments, the population-specific models include more than about 3,000 SNPs, more than about 5,000 SNPs, more than about 10,000 SNPs, more than about 50,000 SNPs, more than about 100,000 SNPs, or more than about 200,000 SNPs. In some embodiments, the plurality of models include models trained on two or more of the population specific genetic datasets. In some embodiments, training the plurality of models is further based on principal components derived from the plurality of individuals. In some embodiments, the plurality of population-specific models include a model for one or more ethnicities selected from the group consisting of: European, African American, Sub-Saharan African, North Africa, LatinX, Central America, East Asian, South Asian, Southeast Asian, West Asian, and Central Asian. In some embodiments, the method further includes deleting the genetic and phenotypic information for the plurality of individuals in each genetic dataset within 30 days of loading the genetic and phenotypic information for the plurality of individuals in each genetic dataset into the cache.

In some embodiments, the user-selected parameters include one or more parameters from the group consisting of: the phenotype of interest, SNPs previously determined to be associated with the phenotype of interest, prior GWAS results for the phenotype of interest, thresholds for dividing the population-specific genetic datasets into training, validation, and test sets, imputation panels, GWAS covariates including sex, age, sequencing platform, and/or principal components, lower limit for SNPs to be included in SNP sets, upper limit for SNPs to be included in SNP sets, a plurality of thresholds for p-values used to determine SNP sets, distance between SNPs in SNP sets, allow list for SNPs, disallow list for SNPs, phenotypic feature to include in model training, type of model to train, hyperparameters for training models, one or more performance metrics for evaluating models, and population-specific ethnicities for which to train a PRS model. In some embodiments, the one or more performance metrics include area under the curve (AUC). In some embodiments, the method further includes: selecting a population-specific SNP set from the plurality of models based on the performance metrics, each population-specific SNP set corresponding to a population-specific genetic dataset; and training the plurality of population-specific based on the corresponding population-specific SNP set.

In some embodiments, the method further includes storing metadata associated with one or more of population-specific models. In some embodiments, the metadata may include one or more of: number of SNPs, SNP selection parameters, area under the curve (AUC) values of the population-specific model, AUC values of the promoted model based on the genetic data and one or more metrics from the group consisting of: age, sex, sequencing platform, and population classifier label, R-squared, relative risk (top vs. bottom and top vs. middle), observed absolute risk (phenotype) difference (top vs. bottom, top vs. middle), and model specification. In some embodiments, the method further includes recalibrating the population-specific models using Platt scaling. In some embodiments, the method further includes: providing a user's data to one of the population-specific models, based on the user's ancestry, to generate a (PRS) score; and generating a user report on the phenotype of interest based on the PRS score. In some embodiments, the user report may include the following outcomes for the phenotype of interest: "Increased Likelihood", "Typical Likelihood", "Not Determined", "Not Applicable."

A non-transient computer-readable medium including program instructions for causing a computer to generate a polygenic risk score (PRS) model to predict phenotypes of a user is provided, the program instructions including: receive user-selected parameters related to a PRS model including a phenotype of interest; select and obtain genetic data for a plurality of individuals based on the user-selected parameters, the genetic data
including data pertaining to a presence or absence of the phenotype of interest in the plurality of individuals; determine a plurality of population-specific genetic datasets from the genetic data based on the plurality of individuals; analyze the population-specific genetic datasets to determine one or more sets of SNPs that are statistically associated with the phenotype of interest for that population-specific genetic dataset, wherein each set of SNPs corresponds with one population-specific genetic dataset; apply SNP filtering criteria to the one or more sets of SNPs to generate a plurality of training SNP sets, wherein the SNP filtering criteria is based at least in part on the user-selected parameters; load into a cache of a computer system genetic and phenotypic information for the plurality of individuals in each population-specific genetic dataset; train a plurality of models using machine learning techniques based at least in part on the genetic and phenotypic information for the plurality of individuals in the cache, the plurality of training SNP sets, and the phenotype of interest; generate one or more performance metrics for each of the plurality of models for each of the population-specific genetic datasets; and determine a plurality of population-specific models based on the one or more performance metrics.

These and other features of the disclosed embodiments will be described in detail below with reference to the associated drawings.

### BRIEF DESCRIPTION OF DRAWINGS

Figure 1 presents a flow diagram of operations for one example embodiment.
Figure 2 presents an illustration of one example embodiment.
Figure 3 presents another illustration of an example embodiment.
Figure 4 presents an illustration of how an interpreter module uses a PRS model to determine a PRS score and provide a report to a user.
Figure 5 presents an example of a modular report according to an example embodiment.
Figures 6-12 provide statistics for an example training a PRS model for LDL-C.
Figure 13 presents an example computer system that may be employed to implement certain embodiments herein.

### DETAILED DESCRIPTION

This disclosure concerns methods, apparatus, systems, and computer program products for determining models used to generate polygenic risk scores ("PRS" or "PGS") for individuals. Genome-wide association studies (GWAS) frequently identify multiple genetic variants (e.g., single nucleotide polymorphisms, or "SNPs") with small to moderate individual impact on the risk for a condition or phenotype. Machine learning methods may be employed to construct statistical models that, given the genetic data and potentially other phenotype data, may generate a PRS score that indicates the risk for a user developing a particular condition or phenotype. Advances in modeling and genome sequencing technology have increased the number of genetic variants that may be studied in a GWAS or included in a PRS model. This results in growing use of PRS models for estimating the risk for a wide range of conditions.

One factor that limits the applicability of PRS models is the size of the training cohort. Very large sample sizes are important both for the GWAS, which identifies genetic variants associated with a condition, and for training a model to estimate the joint contribution of all genetic variants that indicate a correlation with the particular condition. This problem is further exacerbated by different ancestral populations having different combinations of genetic variants. A model developed using data from one ancestry group, e.g., European, does not perform as well when applied to other ancestry groups, e.g., Asian or African.

A PRS Machine can be used to automate and streamline the training of models, track their provenance, and provide users with their individualized PRS predictions via a graphical user interface. The PRS Machine may combine the specifics of a model (e.g., the weights for features) with a user's genetic and phenotypic information to provide back individualized predictions.

Independent of a PRS Machine software release cycle is the operation of a PRS Machine in which high-level details comprising SNP selection (from a Genome Wide Association Study "GWAS"), training phenotype, and additional metadata for cohort definition, acceptance criteria, validation, and more are defined in a PRS-machine repository. On an on-going basis, a researcher may be able to define a model and a PRS Machine fully supports an end-to-end workflow for (re)training, validation, and deployment in the production environment. Models may be defined in a repository, trained on production data, and made available in a performant and scalable web service in the "live" production environment.

Each PRS may include a machine learning model (in some embodiments per chip version, ethnicity, sex, etc.) that produces one of the following outcomes for every user: "Increased Likelihood", "Typical Likelihood", "Not Determined", "Not Applicable" are examples of report outcomes for logistic regression models. "Not applicable" means that a user should not receive a report due to other genetic risk factors. For example, users who are FH+ may not receive any interpretation of the polygenic LDL score. Users who are BRCA+ may not receive any information about their polygenic breast cancer score. The interactions of high penetrance monogenic pathogenic variants and polygenic scores is not well understood, and may confuse the user. Other reasons a model might be "not applicable" for a user: invalid ethnicity, wrong sex, wrong chip version. PRS can be included models built with linear regressions that have numerical report outcomes like quantified risk, predicted BMI, etc.

Each of these PRSes may be trained on individual-level data after hyperparameter optimization based on a model specification checked into the repo on production data within a PRS machine.

### PRS End to End Process Overview

Described herein is an end-to-end pipelined process that enables automated and scalable development and deployment of Polygenic Risk Score (PRS) models delivered to users in the form of streamlined reports. This process may allow for consistency between environments in which models are developed and deployed, reducing and/or eliminating the need to translate or reimplement the core machine learning model implementation between research environments and user environments.

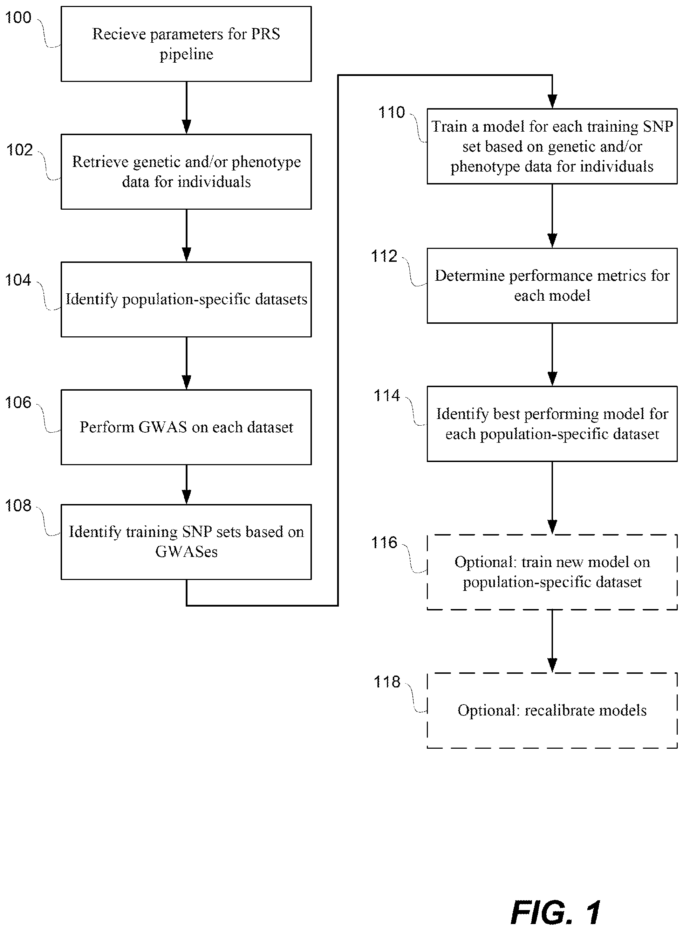

Figure 1 provides a process flow chart for an example embodiment to develop a PRS model for each of various ancestries or populations. In operation 100 parameters for a PRS pipeline may be received. Parameters may define various parts of training a PRS model. For example, the parameters may indicate which phenotype the model is being developed for, how the training cohorts are split into train, validation, and test groups, thresholds for performing a GWAS on a population-specific dataset, etc. In some implementations, the parameters are contained in a specification file. The specification file may by validated to confirm that each parameter has been set. The rest of the process for training a PRS model, such as the process shown in Figure 1, may then be performed based on the parameters in the specification file without further input on the part of a data scientist or other individual to train a PRS model.

The 23andMe database currently has genetic data for greater than 10,000,000 individuals and over three billion phenotypic data points. The methods described herein utilize individual level genetic and phenotypic data for a target phenotype. In order to use the particular individual's data for a target phenotype, the corresponding individual's phenotype states (e.g. absence or presence of the target phenotype or numerical value for the phenotype) needs to be known. For a given target phenotype, the database will contain different numbers of individuals (e.g. Y total individuals) having phenotypic data corresponding to the target phenotype from the over 10,000,000 individuals. Of the Y total individuals with phenotypic data for the target phenotype, can be broken into different population specific subsets of individuals. Typically, the European population makes up the majority of individuals. The number of European individuals in the database with corresponding phenotypic information is typically on the order of 1,000,000 to 3,000,000 or more for the training sets with roughly 100,000-300,000 individuals in each of the test and validation sets. The number of individuals in other populations also varies and is usually on the order of several hundred thousand individuals in the training cohort and on the order of tens of thousands of individuals in the test and validation cohorts.

The user input can include specifying minimum thresholds for the number of cases required to run a population specific GWAS. In some aspects, the minimum number of cases is greater or equal to 5,000 cases, greater than or equal to 6,000 cases, greater than or equal to 7,000 cases, greater than or equal to 8,000 cases, greater than or equal to 9,000 cases, greater than or equal to 10,000 cases, greater than or equal to 15,000 cases, or greater than or equal to 20,000 cases.

The user input can include specifying minimum thresholds for the number of individuals in the validation cohort and test cohort having a known target phenotype status and/or a ratio to apply for the algorithmic determination of the training, validation, and test cohorts. In some aspects, the minimum number of individuals for the test cohort is greater than or equal to 3,000 individuals, greater than or equal to 4,000 individuals, greater than or equal to 5,000 individuals, greater than or equal to 6,000 individuals, greater than or equal to 7,000 individuals, greater than or equal to 8,000 individuals, greater than or equal to 9,000 individuals, greater than or equal to 10,000 individuals, greater than or equal to 15,000 individuals, or greater than or equal to 20,000 individuals. In some aspects, the minimum number of individuals for the validation cohort is greater than or equal to 3,000 individuals, greater than or equal to 4,000 individuals, greater than or equal to 5,000 individuals, greater than or equal to 6,000 individuals, greater than or equal to 7,000 individuals, greater than or equal to 8,000 individuals, greater than or equal to 9,000 individuals, greater than or equal to 10,000 individuals, greater than or equal to 15,000 individuals, or greater than or equal to 20,000 individuals. The minimum number of individuals can be used to determine when there are enough individuals of specific population to form a separate population cohort for GWAS and model training.

In some embodiments the algorithmic determination of the individuals having a known phenotype status for the training, validation, and test cohorts can be received via the user interface as a ratio. For example, the ratio can be provided as a series of 3 numbers, e.g. 8:1:1, corresponding to the training:validation:test cohort ratios, respectively. In some aspects the training cohort can include greater than about 50%, greater than about 55%, greater than about 60%, greater than about 65%, greater than about 70%, greater than about 75%, greater than about 80%, greater than about 85%, greater than about 90%, or greater than about 95%. The validation and test cohorts can include a ratio for the remainder of the individuals having the known phenotype that are not included in the training cohort. For example, the validation and test cohorts can be determined in a 1:1 ratio. In some aspects the ratio between validation:test cohorts can be greater than about 2:1, greater than about 1:1, less than about 1:1, or less than about 1:2 and ratios there between.

A PRS model can be comprised of input features, covariates, model types, hyperparameters, training/test/validation cohorts, threshold criteria, and phenotypes which are predicted. These are defined declaratively so that as a unit, the PRS machine can version each unique PRS model. Because each PRS is defined declaratively, in some cases there is no code that is specially written or tested on a per model basis. The PRS-Machine software may efficiently reason about the inputs for each PRS to bulk load the features. The machine may automatically detect changes to individual PRSes and retrain them. Clients of the machine may use hashes of the PRS definition to distinguish between versions when requesting an inference. Authors can develop and deploy these models without extensive programming expertise or rigorous security audits. The system and methods described herein can automatically generate model definitions based on the latest available GWAS. A clear declarative interface for authoring and modifying PRSes enables the clear separation of roles between software engineers and model authors.

In operation 102 genetic and/or phenotype data for individuals is received. A dataset may be created or accessed comprising genotypic and phenotypic information about a plurality of users. These are users that have consented to research based on their information and who are eligible to be included in research purposes based on the country and region where they live. Genotype information may be gathered by processing an individual's provided sample. Phenotype information may be provided in the form of, e.g., self-reported surveys, family history, imported medical records, biomarkers, data from wearable sensors, and other passive data collection sources.

In operation 104 population-specific datasets are identified. In some implementations, a PRS model may be trained for various populations, including European, African American, Sub-Saharan African, North Africa, LatinX, Central America, East Asian, South Asian, Southeast Asian, West Asian, Ashkenazi, and Central Asian. In some implementations a threshold is set for each population to be identified as a dataset for training a PRS model. As noted above, a large sample size is important to generate useful results from a GWAS. Typically, the number of genetic associations in a GWAS scales on the order of linearly with the sample size of the GWAS. Thus, populations that have a threshold number of case/control individuals may be used for a population-specific GWAS to identify SNPs.

In some implementations each population-specific dataset is further divided into train, test, and validation sets. The use of each group is discussed further herein. Generally, the train sets are used for performing a GWAS to identify relevant SNPs and for training PRS models. The validation sets may be used to determine performance metrics for trained models to evaluate each model, adjust hyperparameters, and potentially training or re-training PRS models. The test sets may be used to generate final performance metrics for PRS models that are used in production, where the final performance metrics may be used to, e.g., compare a newly trained model against a model currently in production. In some implementations there are thresholds for dividing a population-specific dataset into train, validation, or test sets. For example, a small dataset may only be used as a test set, while a larger dataset may be divided into a test set and validation set, but not a train set.

In operation 106 a genome wide association study (GWAS) may be performed for a particular phenotype to be studied. A GWAS may be run on all of the individuals in the dataset, or a subset of individuals based on various filtering criteria. In some implementations, the result of a GWAS is the identification of single nucleotide polymorphisms (SNPs) that are statistically associated with the phenotype of interest. The identified SNPs exhibit a strong correlation for the particular phenotype.

In operation 108 a plurality of training SNP sets are identified based on the GWAS results. In some implementations, where there are multiple GWAS results, the results of each GWAS may be combined. In some implementations multiple GWAS results are available as a result of running a GWAS on train sets for different populations. In some implementations, external GWAS results may be received and combined as well, for example GWAS results available from other researchers. This combination may be performed by an inverse weighting to combine results from each GWAS, sometimes referred to as a meta-analysis. The resulting combined set of SNPs may then be filtered based on quality control metrics to determine a plurality of SNP sets that are used for training. In some embodiments, the SNPs may be filtered prior to running a GWAS, and then filtered a second time after the GWAS. In some embodiments, a plurality of SNP sets are generated by variant selection criteria.

In operation 110 the plurality of SNP sets may be used to train one or more machine learning models to generate a PRS score for an individual for the particular phenotype. Each model may be trained based on various features and/or hyperparameters. Non-genetic features used in training may include age, sex, age*sex, age², age²*sex, and principal components derived from one of the populations (e.g., the European ancestry population). Other phenotypic information can also be included in the features and/or hyperparameters, including other phenotypes, family history, environmental factors, etc. In some implementations a model is trained based on each population having a train dataset. For example, if there are three populations having a training set, and 100 different sets of SNPs/features/model hyperparameters, 300 models may be trained.

In some implementations the models are trained based on the individual level data of individuals in the train dataset. This is advantageous over training models based on the summary statistics of a GWAS alone, as the model does not have to rely on the summary statistics that result from the GWAS (GWAS results typically include the SNP, phenotype, odds ratio, minor allele frequency (MAF), and p-value, but do not include the call at every SNP for every individual). Instead, the model may learn based on the underlying individual level data. Furthermore, in some implementations the PRS models are also trained based on the phenotype data of each individual, which may include additional information beyond the phenotype of interest for which the PRS model outputs a score.

In operation 112 performance metrics are determined for each model using the validation datasets. In some implementations every trained model is evaluated on each validation set. Each model may be evaluated, compared, and optionally recalibrated. In some embodiments, the model with the best performance metrics may then be validated. In some embodiments, the metadata associated with each model may be stored. One of the models may then be used for generating PRSes for a user.

In operation 114 the best performing models for each population-specific dataset are identified. In some embodiments the particular SNP set, other features, and/or model hyper-parameters are identified. In some embodiments, the performance metrics may include: AUC (optionally based on genetic data only or genetic data and other covariates, e.g., age, sex, etc.), relative risk (top v. bottom and/or top vs. middle), and observed absolute risk (phenotype) difference (top vs. bottom, top vs. middle). In some implementations the best performing model is identified based on having the highest AUC value. Generally, a goal of a model is to maximize these metrics to best stratify the population.

Operation 116 is an optional operation to train a new model for one or more of the population-specific datasets. In some implementations the new model is trained on the train and validation sets or the train, validation, and test sets, rather than just the train datasets. In some implementations the new model is trained based on the SNP set, feature set, and model hyper-parameters identified in operation 114. For example, a plurality of candidate models may be initially trained on a European training set and then validated on a smaller Hispanic/LatinX validation set. The parameters for the model that performed the best on the Hispanic/LatinX set may then be used to train a new model based on a combination of one or more of the European training, validation, and test sets and optionally the Hispanic/LatinX validation set.

After a model is trained to provide a PRS it may be used in production to determine PRS scores for users. The model is called and takes as an input the user's data and outputs a PRS. In some embodiments, the model has predicate conditions for use, such as sex, population classifier label, or age, such that a particular model is used to generate a PRS based on the user's data for the predicate conditions. The PRS is then provided to an interpreter module that creates a customer report. An interpreter module takes in a user's PRS and may output a qualitative result (i.e., "Typical" or "Increased" likelihood) and/or a quantitative likelihood estimate (i.e., 28% chance of X by age X). The interpreter module provides a complete report experience for a user. An interpreter module is separate from a model providing a PRS, allowing for separate iteration of the model or the interpreter module without impacting the other component.

As noted above, a particular challenge for PRS models is that different PRS models perform better for different populations. In particular, while there is a large amount of genotype data for European populations, there may be insufficient data for non-European ancestries. To address this, PRS models for non-European ancestries, or populations without a sufficient sample size, may be generated in various ways. Overall, no one method works for every phenotype-ancestry combination. The specific method used for each ancestry group may be considered a hyperparameter and optimized on a case-by-case basis. Furthermore, as noted above, validation and testing may be done in ancestry-specific datasets to avoid overestimation of performance metrics.

One method that may be used for phenotypes and ancestries with relatively large sample sizes is to conduct a separate GWAS for each group, and ancestry-specific PRS models are created from these ancestry-specific GWAS. However, for many phenotypes there are insufficient individuals or survey responses to run sufficiently powered GWAS independently for all ancestry groups.

A second approach is to leverage information from the European GWAS to boost power for the non-European GWAS. A meta-analysis may be used to combine information for each SNP across ancestries and generate a PRS model leveraging training sets comprised of multiple ancestry groups (while controlling for population structure using genomic principal components).

A third approach is to run a GWAS and train a PGS using European-ancestry data, with model hyperparameters optimized based on performance in a validation dataset consisting of data from the non-European ancestry group.

Fourth, in some implementations the European PRS model may be used for non-European ancestry groups.

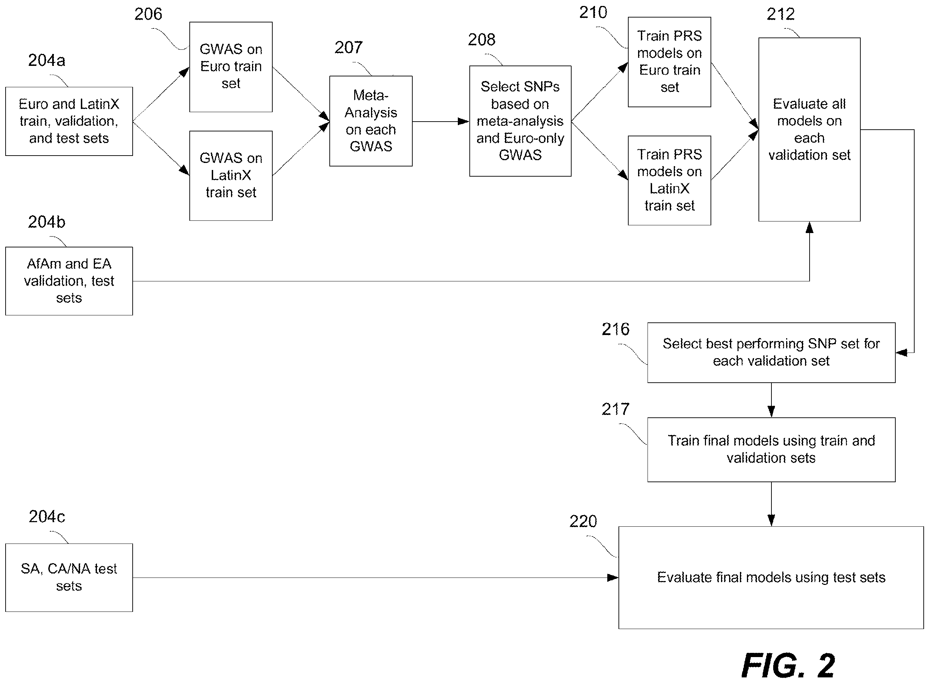

Figure 2 presents an example series of operations for training a model based on the flowchart of Figure 1. Starting in blocks 204a-c, cohorts are identified for train, validation, and test sets. Block 204a includes train, validation, and test sets for European and LatinX populations, block 204b includes validation and test sets for African American and East Asian populations, and block 204c includes test sets for South Asian and Central Asian/North African populations. The difference between blocks 204a-c is the number of individuals that qualify for the cohort selection. While there are a sufficient number of individuals of European and LatinX ancestry to exceed a threshold and divide the population-specific datasets into train, validation, and test sets, the number of African American, East Asian, South Asian, and Central Asian/North African individuals does not exceed the threshold.

In block 206 a GWAS is performed on the European training set and the LatinX training set, respectively. It should be understood that while two GWAS are shown in Figure 2, a GWAS may be performed on each population that has a sufficient number of individuals to exceed a threshold for having a test set. The result of each GWAS may include a set of SNPs and associated p-values for the phenotype of interest.

In block 207 a meta-analysis is performed to combine the results from each GWAS. As noted above, the combined set of SNPs may result from an inverse-weight of the results from each GWAS or other suitable techniques.

In block 208 a plurality of SNP sets are generated based on the meta-analysis and the European GWAS results. As the European dataset is typically the largest dataset, the European GWAS may be used to identify SNPs that are applicable to other populations. Variations on filtering criteria may be applied to the combined set of SNPs to generate the plurality of SNP sets, such as varying the p-value thresholds, linkage disequilibrium distance, SNP windows, etc. Each SNP set may also include hyper-parameters for how the model training is to proceed, including the learning technique, covariates, principal components, etc.

In block 210 a model is trained for each SNP set on each training set of data. Each SNP set is used to train a model on the European training set and on the LatinX training set.

In block 212 each trained model is evaluated on each validation set. As noted above, block 204b represents populations that do not have a training set but do have a validation set. Thus, each model is validated on the validation sets for African American and East Asian populations. The result of block 212 is a performance metric, such as AUC, for each model for each validation set. In block 216 the best performing SNP set (along with other features and hyper-parameters) is selected for each validation set/population. In some implementations this is the SNP set having the highest AUC metric.

In block 217 the final models are trained for each population. In some implementations, the final model is trained on the validation set and testing set for that population, for example the LatinX population. In some implementations, the final model for a particular ancestry is trained on the train and validation set for a different ancestry, for example the East Asian final model may be trained on the train and validation set for the European ancestry, but using the SNP set and other feature/hyperparameters that performed the best for the East Asian validation set. In some implementations the East Asian validation set may also be combined with the European train and validation set to train the final model.

Finally, in block 220 each final model is evaluated using the population-specific test set. For populations that did not have a validation set, such as those in block 204c, the European final model is evaluated on the test set for those populations. In some embodiments, the European final model is used in production for those populations lacking sufficient genetic and/or survey data to form a validation set. The final metrics may then be stored and used for, e.g., comparing the current model against a new model that may be later trained.

### PRS End to End - Full Flow

### 1. Data Collection

As noted above, datasets used for training a model include users who have consented to participate in research and have answered survey questions required to define the phenotypes of interest. Data collection may involve collecting genomic samples from individuals and sequencing the samples, as well as collecting survey responses or other phenotypic data from individuals. In some embodiments, datasets are based on males and females between the ages of 20 and 80. In some embodiments, datasets are filtered to remove individuals with identity-by-descent of more than about 700 centimorgans, with the less rare phenotype class removed preferentially. Individuals may also be grouped into various populations, e.g., Sub-Saharan African/African American, East/Southeast Asian, European, Hispanic/Latino, South Asian, and Northern African/Central & Western Asian datasets. In some embodiments, a model may be trained on one ethnic group, e.g., European, and then used for another ethnic group.

In some embodiments, individuals may also be grouped based on the genotyping technology used to determine an individual's genotype. In some embodiments samples are run on one of three Illumina BeadChip platforms: Illumina HumanHap550+ BeadChip platform augmented with a custom set of ~25,000 variants (V3); the Illumina HumanOmniExpress+ BeadChip with a baseline set of 730,000 variants and a custom set of ~30,000 variants (V4); and the Illumina Infinium Global Screening Array (GSA), consisting of 640,000 common variants supplemented with ~50,000 variants of custom content (V5). Samples with a call rate of less than 98.5% may be discarded.

In some embodiments, the dataset may include imputed genomic data or functionally aggregated data. In certain embodiments, some alleles are imputed to an individual's genetic composition even though the genotype information pertaining to the allele or its polymorphism was not directly assayed (i.e., not directly tested using a genotyping chip or other genotyping platform) for the individual. By imputation, the individual is deemed to have the specific genetic variant. Examples of imputation techniques include statistical imputation, Identity by Descent (IBD)-based imputation, and a combination thereof. A discussion of some aspects of imputation appear in US Patent Application Publication No. 2017-0329901, published November 16, 2017. The imputed genetic data can sometimes be referred to as dosages with the imputed variants stored as a probability of the imputed variants being present in the individual.

Examples of polymorphisms that may have imputed alleles include Single Nucleotide Polymorphisms (SNPs), Short Tandem Repeats (STRs), and Copy-Number Variants (CNVs). Although SNP-based genotype data is described extensively below for purposes of illustration, the technique is also applicable to other forms of genotype data such as STRs, CNVs, etc.

### Statistical Imputation

In some embodiments, imputation includes statistical imputation. A statistical model such as a haplotype graph is established based on a set of reference individuals with densely assayed data. Sparsely assayed genotype data of a candidate individual (i.e., an individual whose genotype corresponding to a polymorphic variant of interest (VOI) site is not directly assayed) is applied to the statistical model to impute whether that individual possesses the VOI.

To perform statistical imputation, a reference data set of densely assayed data is used to construct a statistical model (e.g., a haplotype graph) used to determine likely genotype sequences for the candidate individuals. In some embodiments, full genome sequences are used. The number of reference individuals in the densely assayed reference data set may be fewer than the number of candidate individuals in the sparsely assayed data set. For example, there can be 100,000 or more individuals in the sparsely assayed data set, but only 1000 in the densely assayed data set.

In operation, a likely genotype sequence is identified based on the candidate individual's genotype data and the statistical model. In some embodiments, at least a portion of the sparsely genotyped data (e.g., a portion that overlaps the VOI location) is compared with paths on the haplotype graph to find a most likely path (i.e., a likely genotype sequence).

Other types of statistical imputation can be used including using imputation panels assembled based on fully sequence data for a plurality of individuals. The full sequence data can be from publicly available datasets such as the International HapMap, 1000 genomes project, and the like alone or in combination with proprietary sequence data from 23andMe research participants.

### Identity by Descent (IBD)-based imputation

In some embodiments, imputation includes identifying IBD regions between a proband and a candidate individual. IBD-based imputation does not require a reference set of densely assayed genotype data.

Because of recombination and independent assortment of chromosomes, the autosomal DNA and X chromosome DNA (collectively referred to as recombining DNA) from the parents are shuffled at the next generation, with small amounts of mutation. Relatives (i.e., people who descended from the same ancestor) will share long stretches of genome regions where their recombining DNA is completely or nearly identical. Such regions are referred to as "Identity (or Identical) by Descent" (IBD) regions because they arose from the same DNA sequences in an earlier generation. In some embodiments, individuals in a database that share a variant-overlapping IBD region with the proband are identified. A variant-overlapping IBD region is an IBD region that overlaps the location where the VOI is found.

In some embodiments, the determination of IBD regions includes comparing the DNA markers (e.g., SNPs, STRs, CNVs, etc.) of two individuals. The standard SNP based genotyping technology results in genotype calls each having two alleles, one from each half of a chromosome pair. As used herein, a genotype call refers to the identification of the pair of alleles at a particular locus on the chromosome. The respective zygosity of the DNA markers of the two individuals is used to identify IBD regions. In some cases, IBD identification can be performed using existing IBD identification techniques such as fastIBD.

When two individuals have opposite-homozygous calls at a given SNP location, it is very likely that the region in which the SNP resides does not have IBD since different alleles came from different ancestors, and the region is not IBD. If, however, the two individuals have compatible calls, that is, both have the same homozygotes, both have heterozygotes, or one has a heterozygote and the other a homozygote, there is some chance that at least one allele is passed down from the same ancestor and therefore the region in which the SNP resides is IBD. Further, based on statistical computations, if a region has a very low rate of opposite-homozygote occurrence over a substantial distance, it is likely that the individuals inherited the DNA sequence in the region from the same ancestor and the region is therefore deemed to be an IBD region.

### 2. Model Development

### Cohort Identification

In order to develop a PRS model for a phenotype of interest an analysis cohort may be determined - a list of individuals to be used in training, validation and testing of one or more machine learning models. The analysis cohort may be generated by filtering the dataset using one or more of the following parameters:
a. Research consent status and eligibility
b. Filter for individuals by missing SNP values
c. Filter for relatedness, and bias for cases with more rare phenotypes
   i. This is a measure of maximum relatedness between two participants. This is defined as no more shared IBD segments summing to a total length greater than about 700cm and when choosing between related individuals, bias towards choosing the cases with more rare phenotypes.
d. Additional filtering capabilities are also of interest. These may include:
   i. Minimum and maximum ages, e.g., about 20 and about 80 years old.
   ii. Specific sequencing platforms, e.g., V3, V4, or V5 as described above.
   iii. Specific population classifier labels
   iv. Single or both sexes
   v. A custom proportion of train/validation/test

The analysis cohort may then be split into training, validation, and test sets using a 70:20:10 or 80:10:10 split (or a proportion defined as an advanced filtering feature above). In some embodiments, a different split may be used. In some embodiments, multiple analysis cohorts may be generated by using different filtering parameters. In some embodiments, an analysis cohort may be generated for specific populations. The training, validation, and test sets may also be filtered to reduce the chance of related individuals being in different sets.

In some embodiments a threshold is used to determine whether to split a cohort for a particular population into training, validation, and test sets. If there is an insufficient number of individuals of a particular ancestry who have provided information as having the phenotype of interest, then a model trained on such a group may not provide better predictions for that ancestry than another ancestry having a larger sample size. Furthermore, there may also be insufficient individuals to validate the model using a dataset for that population. Thus, in some embodiments, the dataset may only be divided into a validation and test cohort if a first threshold number of individuals in that dataset have the phenotype of interest. Furthermore, in some embodiments the dataset may only be divided into a training, validation, and test set if a second threshold number of individuals in that dataset have the phenotype of interest, where the second threshold is higher than the first threshold. In some embodiments, if a dataset does not have a number of individuals exceeding either threshold it may be labelled as a test set. In some embodiments, the first threshold may be at least about 8,000, at least 10,000, or at least about 20,000 individuals of that ancestry that have the phenotype of interest. In some embodiments the second threshold may be at least about 50,000, at least about 80,000, at least about 100,000, or at least about 200,000 individuals of that ancestry that have the phenotype of interest.

The IDs for the training/validation/test sets for that given phenotype and their metadata (see below) may then be cached and stored in perpetuity for use and reference downstream (ie: saved in a file accessible to GWAS and PRS machine). The metadata for an analysis cohort may include: when the cohort was assembled, what time, and what analysis was associated with that cohort at that time. This metadata may be a feature carried through the PRS development pipeline.

After identifying an analysis cohort of individuals for which phenotype data is known as to whether each of the individuals has or does not have the desired phenotype, the cohort can be separated into cases (those with the target phenotype) and controls (those without the target phenotype). The analysis cohort can be split into training, validation, and test sets. As discussed above, the GWAS may be run on the training set data. Importantly, in some embodiments, the GWAS may not be run on the validation or test sets. The GWAS identifies SNPs that statistically correlate with the studied phenotype. In some embodiments, prior to running the GWAS, the training set data may be filtered to remove some SNPs from consideration in the GWAS according to various QC metrics. For example, some SNPs are 99.9999% 'A' in a population, and thus are not useful for predicting within that group. Other SNPs may be blocked for, e.g., not calling at a sufficiently high accuracy, and thus would not be used in a model. In some cases, the training set may also be filtered by various covariates, including age, sex, population classification, population specific principal components (PCs), sequencing platform, and custom phenotypes (e.g., BMI, age^2, age^4, etc.).

### SNP Set Generation

The SNP sets used for training a PRS model may be determined from the results of one or more GWAS. In some implementations, a product scientist may select a phenotype to run a GWAS on via a user interface or specification file. Covariates may also be selected for the GWAS via the user interface (like Age, Sex, Population Classification, Population specific principal components (PCs), Platforms, Custom phenotypes (of any type, this can include BMI, Age^4, etc.) In some embodiments, covariates may be used to filter which individuals are included in a training cohort that a GWAS is run on. In other cases, covariates may be used as part of the GWAS to determine statistical correlations.

Then, in some embodiments, a GWAS is run for that chosen phenotype and its related *training* cohort. The results may be stored in a database and accessible to downstream systems in Production and the R&D environment for analysis.

The output of a GWAS includes a list of SNPs and statistical correlations with the phenotype being studied. After the GWAS, the PRS machine then takes all SNPs over a certain p-value from the GWAS results table based on the specified criteria received via the user interface. In some implementations, a list of SNPs and statistical correlations may be received without running a GWAS as part of the model training process, for example using a previously run GWAS. In some implementations, multiple GWAS results may be used, subject to a meta-analysis that combines results across different GWAS, using e.g., inverse weighting.

### SNP Filtering

The result of the GWAS (or meta-analysis of multiple GWAS) includes a list of SNPs and associated p-values. This list of SNPs may be subject to additional filtering, including by p-value.

The first filtering step is to use QC filtering. QC filtering may include referencing allow lists and/or block lists. In some embodiments, SNP quality metrics may be used to filter the list of SNPs, including no call rates, false positives, or false negatives. In some embodiments, SNPs that don't vary across every population may be filtered out. In some embodiments, this step may be performed prior to running the GWAS, and if so may not be repeated after running the GWAS.

A second filtering step may include distance pruning. The goal of this stage of filtering is to remove nearby, likely correlated SNPs with lower effect sizes. This may be accomplished by generating hundreds of different sets of SNPS based on all combinations of different parameter values. The different sets of SNPs may then be used to train individual models. The performance of these hundreds of models are compared to determine which model (and which SNPs) result in the most accurate model.

The different parameter values used to generate different sets of SNPs include p-value and window size. P-value is a measurement of how likely a disease-associated variant is due to random chance and is an output of the GWAS. Window size is a range (in base pairs) that is considered when applying distance pruning.

In some embodiments, linkage disequilibrium (LD) pruning may also be used to generate different SNP sets. LD pruning may be based on p-value, window size, and a threshold for correlation (r2). R2 values can be referenced or generated in a number of ways: referenced to a publicly available or developed LD panel, generated as a reference a static LD panel (e.g., 1 LD panel for about 100 phenotypes), or generated and referenced to 1 LD panel per model. Distance pruning: There are 2 parameters that vary with genetic distance pruning: p-value, window size. P-value is the measurement of how likely a disease-associated variant is due to random chance, which is an output of the GWAS. Window size is the range (typically in basepairs) that is considered when applying distance pruning. This filtering criteria is specified via the user interface. LD (linkage disequilibrium) pruning: There are typically 3 parameters that vary with LD pruning: p-value, window size, threshold for correlation (r2). R2 describes the pairwise relationship between all nearby variants. In some embodiments, an elasticnet may be used to filter SNPs. Using elasticnet can eliminate the need for hundreds of SNPsets / models trained. Although the above
steps are illustrated for performing a GWAS, other techniques can also be used for determining the SNPs to use for model training. For example, neural networks and other machine learning techniques can be used.

### PRS Training

Each SNPset is then used with the training cohort to train a machine learning model. In addition to the SNPset, the following features may be specified for each model. In some embodiments these features may be specified in a particular specification file that defines the PRS model training process:
a. Variants (narrowed down from filtering activities described herein)
b. Model fitting method (ie: logistic). Other Fitting methods can include regression algorithms (eg, generalized linear models), regularized algorithms (eg, ridge regression, LASSO, and elastic net), clustering algorithms (eg, k-means), bayesian models, and neural networks.
   i. Model parameters (ie: class_weight, max_iterations, penalty)
c. Phenotype data
   i. Age
   ii. Sex
   iii. Phenotypic formula
   iv. Phenotype of Interest
   v. Related specifications (ie: min/max age)
   vi. medical records
   vii. biomarkers
   viii. data from wearable sensors
d. Principal Components
e. Mean dosages for missing values - these can be gathered in a number of ways
   i. Referenced from another source
   ii. Looking at the Research Env and calculating the mean dosage
   iii. Use the training samples to calculate the mean dosages
f. Cohorts file (SNP selection uses the validation set)
g. Model / Ethnicity specification (if multiple models per report - this information is currently housed in the "interpreter spec" file)
h. Baseline prevalences (for quant result generation)
i. Distribution thresholds (for quant result generation)
j. Performance metrics (for validation)

In order to scalably train all the models in parallel, the data used for training (ie: union of N variant sets and phenotype values for all individuals in training, validation, test cohorts) may be collected and cached locally.

The PRS machine may then perform parallelized training on the order of 10s or hundreds of models or more, one for each SNPset defined during distance pruning based on the user specified criteria in the user interface. All metrics may be tracked and stored. In some cases, each model may be trained on a different SNPset and have the same features specified above. In some cases, each model may be trained on a different SNPset and features may not be the same across all model training.

### Model Training and Output Predicates

The models described herein may include different predicates and criteria for who is used to train the model and for who can receive scores in the model. For example, most models may be trained on consented people over a certain age with a well defined self-report for the phenotype of interest. However, predictions from a PRS model may be provided to a different (typically broader) set of individuals using different predicates. The set of people eligible to be included in the training, and the set of people eligible to receive results are defined by different sets of predicates.

There are also multiple sources for phenotypes that could all be combined for the self reported information from a user. For example: self report of X condition, family history of X, medical records including X, response to X medication, passive data collection indicating X, and others. Logic can be used to determine what the expected phenotype is from a series of different responses related to the phenotype of interest. Depending on the type of the specific self reported information for the phenotype of interest the strength of the self report can be determined or estimated. If the self report is determined to be accurate information for the presence of absence of X phenotype then the individual can be included in the cohorts used for GWAS and model building. Conversely if the determination of the absence or presence of X phenotype in the individual is uncertain from the self reported information then the individual may be excluded from the cohorts used for GWAS and model building.

Phenotypes that can be predicted by the prediction machine learning models include disease as well as non-disease related traits, such as height, weight, body mass index (BMI), cholesterol levels, etc. The types of predictions include but are not limited to the probability of a disease occurring over the course of an individual's lifetime, the probability of a disease occurring within a specific time frame, the probability that the individual currently has the disease, odds ratios, estimates of the value of a quantitative measurement, or estimates of the distribution of likely measurements.

A phenotype model generator and model applicator can be implemented as software components executing on one or more general purpose processors, as hardware such as programmable logic devices and/or Application Specific Integrated Circuits designed to perform certain functions or a combination thereof. In some embodiments, these modules can be embodied by a form of software products which can be stored in a nonvolatile storage medium (such as optical disk, flash storage device, mobile hard disk, etc.), including a number of instructions for making a computer device (such as personal computers, servers, network equipment, etc.) implement the methods described in the embodiments of the present invention. The modules may be implemented on a single device or distributed across multiple devices. The functions of the modules may be merged into one another or further split into multiple sub-modules. In some embodiments the model generation and model applicator can be implemented in a cloud computing platform.

A machine learning model platform is configured to use individual level information of a significant number of customers to build and optionally validate one or more machine learning models for phenotype prediction. In some embodiments the individual level information may be loaded into a cache and used for training all models in a parallelized process. In some embodiments this may improve the efficiency of the training process by loading individual user data once and then training all models.

In some embodiments, the individual level information is retrieved from one or more databases. The individual level information may include genetic information, family history information, phenotypic information, and environmental information of the members.

In some embodiments, the family history information (e.g., a relative has a particular disease and the age of diagnosis) and the environmental information (e.g., exposure to toxic substances) are provided by the members, who fill out online questionnaires /surveys for themselves. In some embodiments, some of the family history information and environmental information is optionally provided by other members. For example, some online platforms allow members to identify their relatives who are also members of the online platforms and make a connection with each other to form family trees. Members may authorize other connected relatives to edit the family history information and/or environmental information. For example, two members of the network-based platform may be cousins. They may authorize each other to fill out parts of their collective family history, such as the medical history of grandparents, uncles, aunts, other cousins, etc. The genetic information, family history information, and/or environmental information may also be retrieved from one or more external databases such as patient medical records.

In some embodiments, modeling techniques (e.g., machine learning techniques such as regularized logistic regression, decision tree, support vector machine, etc.) are applied to all or some of the member information to train a model for predicting the likelihood associated with a phenotype such as a disease as well as the likelihood of having a non-disease related genotype such as eye color, height, etc. In some embodiments, the models are derived based on parameters published in scientific literature and/or a combination of literature and learned parameters. The model may account for, among other things, genetic information and any known relationships between genetic information and the phenotype.

In some embodiments, the predicted outcome is age dependent. In other words, the predicted outcome indicates how likely the individual may have a particular disease by a certain age/age range.

Some aspects of trained models for phenotype prediction are presented in U.S. Patent Application Publication No. 20110130337, titled "Polymorphisms Associated with Parkinson's Disease," and filed November 30, 2010, and in U.S. Patent Application Publication No. 20170329904, titled "DATABASE AND DATA PROCESSING SYSTEM FOR USE WITH A NETWORK-BASED PERSONAL GENETICS SERVICES PLATFORM," and filed May 10, 2016.

In some embodiments, a logistic regression technique is used to develop the model. In this example, a subset of the customers are selected as training data and the remaining customers are used for validation and test sets.

In one example where logistic regression is performed, for each customer used in a training set, the genetic and environmental information is encoded as a multidimensional vector. Many possible encoding techniques exist. One example of a specific encoding technique is to include the number of copies of risk alleles for each SNP (0, 1, or 2) as separate entries in the vector, the presence or absence of the phenotype in any relative (0 = no, 1 = yes), and the presence or absence of various environmental factors (0 = no, 1 = yes, per environmental factor). Each of the elements of the vector may be referred to as "features." For notational convenience, the multidimensional vector for the i-th customer may be denoted as **x**^{(*i*)} = (*x*_{*i*,1}, *x*_{*i*,2}, ..., *x_{i,n}*). Here, *n* represents the number of encoded features, and *x_{i,j}* represents the j-th encoded feature for the i-th example. Let *m* denote the number of examples in the training set, and let **y** = (*y*⁽¹⁾, *y*⁽²⁾, ..., *y*^{(*m*)}) denote an encoding of the phenotypes for each individual in the training set (*y*^{(*i*)} = 1 indicates that the *i*-th individual reported developing the disease, whereas *y*^{(*i*)} = 0 indicates that the *i*-th individual did not report developing the disease).

In the logistic regression example, a model may have the form: $P \left(y=\left.1\right|x; w,b\right) = 1 / \left(1+exp\left(-wTx-b\right)\right)$ (1).

Here, **x** corresponds to an *n*-dimensional vector of encoded features, and *y* is the encoded phenotype. The parameters of the model include *b* (a real-valued intercept term) and **w** = (*w*₁, *w*₂, ..., *wₙ*) (an *n*-dimensional vector of real-values). The notation **w**^{T}**x** is taken to mean the dot product of the vectors **w** and **x** (i.e., ∑*ⱼ*=_{1,...,}*ₙ wⱼ xⱼ*). The exp() operator refers to exponentiation base e. For any vector **x,** the logistic regression model outputs a value between 0 and 1 indicating the probability that an individual with encoded features **x** will report having developed the phenotype such as a disease (i.e., *y =* 1).

In the logistic regression example, the parameters of the model **(w** and *b*) are chosen to maximize the logarithm (base e) of the regularized likelihood of the data; this quantity, known as the regularized log-likelihood, is specified as follows: $L \left(w, b\right) = \sum i = 1 , … , m log P \left(\left.y\left(i\right)\right|x\left(i\right); w,b\right) - 0.5 C w T w$ (2).

Here, *C* is a real-valued hyperparameter that is chosen via cross-validation (as described below). The first term of the objective function is a log-likelihood term that ensures that the parameters are a good fit to the training data. The second term of the objective (i.e., 0.5 **w**^{T}**w**) is a regularization penalty that helps to ensure that the model does not overfit. The hyperparameter *C* controls the trade-off between the two terms, so as to ensure that the predictions made by the learned model will generalize properly on unseen data.

In the logistic regression example, a cross-validation procedure may be used to select the value of the hyperparameter *C*. In this procedure, the parameters of the model (**w** and *b*) may be fit by maximizing the objective function specified in equation (1) for multiple values of *C* (e.g., ... 1/8, 1/4, 1/2, 1, 2, 4, 8, ... ) using data from the training set (e.g., member data for members 1-30,000). For each distinct value of *C*, the process obtains a parameter set, which is then evaluated using a validation objective function based on the validation set (e.g., member data for members 30,001-40,000). The parameters (and corresponding value of C) which achieve the highest validation objective function are returned as the optimal parameters (and hyperparameter) for the model. For this example, a reasonable validation objective function is the following: $L ′ \left(w, b\right) = \sum i = m + 1 , … , M log P \left(\left.y\left(i\right)\right|x\left(i\right); w,b\right)$ (3).

Here, **x**^{(*m* + 1)} through **x**^{(*M*)} correspond to the multidimensional vectors of features for the validation data. Note that the validation objective function does not include a regularization term, unlike the objective function (2).

In some embodiments, the data set is divided into several portions, and training and validation are repeated several times using selected combinations of the portions as the training sets or validation sets. For example, the same set of information for 40,000 members may be divided into 4 portions of 10,000 members each, and training/validation may be repeated 4 times, each time using a different set of member information for 10,000 members as the validation set and the rest of the member information as the training set.

In some embodiments, a decision tree is generated as the model for predicting a phenotype. A decision tree model for predicting outcomes associated with a genotype can be created from a matrix of genotypic, family history, environmental, and outcome data. The model can be generated with a variety of techniques, including ID3 or C4.5. For example, using the ID3 technique, the tree is iteratively constructed in a top-down fashion. Each iteration creates a new decision junction based on the parameter that results in the greatest information gain, where information gain measures how well a given attribute separates training examples into targeted classes. In other cases, the structure of the decision tree may be partially or completely specified based on manually created rules in situations where an automated learning technique is infeasible In some embodiments, the decision tree model is validated in the same way as the logistic regression model, by training and evaluating the model (retrospectively or prospectively) with a training set of individuals (e.g., members 1-30,000) and an independent validation set (e.g., members 30,001-40,000).

**In** some embodiments, the model determination process accounts for genetic inheritance and the correlation of genetic information with family history information. There are various cancer studies showing that certain mutated genes are inherited according to Mendelian principles and people with mutations in these genes are known to be at significantly higher risk for certain types of disease (such as familial prostate cancer). In other words, possession of such mutated genes and having family members that have the disease are highly correlated events. The model, therefore, should account for such correlation.

### Benefits of Using Models Trained on Imputed Data

As noted above, in some embodiments a model may also be trained on imputed data in addition to what is directly assayed on the genotyping chip. The use of imputed data gives a richer dataset over using genotype data only. For example, the number of assayed variants on a genotype chip can be on the order of around 1,000,000 variants. With imputation the number of genetic variants can be orders of magnitude greater. The current imputation panel provides greater than 50,000,000 variants. In some cases the imputation panel can provide greater than 55,000,000 variants, 60,000,000 variants, 75,000,000 variants, 85,000,000 variants, 100,000,000 variants, 110,000,000 variants, 120,000,000 variants, 130,000,000 variants, 140,000,000 variants, or 150,000,000 variants.

Training the PRS models described herein on imputed variants allows for generating models with additional features, such as a greater number of variants/SNPs. The use of additional variants/SNPs in the models improves the model performance (such as by increasing the AUC) as more genetic signals are captured by a model having a greater number of variants.

Some PRS models have been made based on publicly available summary statistics capped at 10,000 SNPs/variants from GWAS that have been made publicly available. 23andMe's T2D model has less than 1,300 SNPs in it. The use of imputed data and the methods described herein allow for building models with much larger feature sets that can still be quickly calculated on demand. In some cases the models have greater than 3,000 SNPs, greater than 5,000 SNPs, greater than 10,000 SNPs, greater than 25,000 SNPs, greater than 50,000 SNPs, greater than 100,000 SNPs, greater than 200,000 SNPs, greater than 250,000 SNPs, greater than 300,000 SNPs, greater than 400,000 SNPs, greater than 500,000 SNPs, greater than 600,000 SNPs, greater than 700,000 SNPs, greater than 800,000 SNPs, greater than 900,000 SNPs, greater than 1,000,000 SNPs, greater than 2,000,000 SNPs, greater than 3,000,000 SNPs, greater than 4,000,000 SNPs, and greater than 5,000,000 SNPs.

Yet another benefit of using imputed data is that the imputed user genetic data is agnostic to the genotyping chip that was used to assay the user's genotype. An additional advantage of using imputed dataset allows for standardization between different chip versions, such as V1, V2, V3, V4, and V5. Imputation of genetic data assayed on V1, V2, V3, and V4 chips allows for those individuals to be included in the model building techniques described herein. It can be cumbersome to generate different models based on the different SNPs that are assayed on different genotype chips. Using imputed data also makes it easier to compare the model performance between different models as no conversion is necessary to account for the inclusion of different variants on different genotyping chips.

### Benefits of Training Models on Individual Level Data

There are a number of benefits with building models based on individual level data instead of GWAS summary statistics. Raw genotype and phenotype data are both needed in large numbers in order to build a model based on individual level data, which poses a problem for institutions that do not have access to such data. Using individual level data is not feasible for many as they do not have access to raw data for a sufficient number of individuals. In addition running machine learning algorithms on such big datasets can be computationally intensive and associated with a high computation cost that is not practical for many.

When using GWAS summary statistics the data used usually includes variant effect size and standard error estimates from GWASs, sample size, and an LD panel that describes the correlation between genetic variants. The intention behind this approach is to use the GWAS summary statistics and associated data to approximate the training process of using individual level data with statistical algorithms. However, there are a number of disadvantages with this approach since it is an approximation of training with raw data. The prediction accuracy is expected to be much lower given the many rough assumptions and approximations that are required. For example the distribution of effect sizes across the genome, which could be violated and lead to bad PRSs such as when the summary data being used do not match one another. For example, the LD panel does not correctly reflect the correlation between markers in the GWAS.

Once the individual level data is gathered and quality controlled, machine-learning models can be applied to the dataset as described herein to explore the relationship between the variants and traits and to then make predictions on the phenotype given the genotypes. Since raw data is used in the training process, the PRSs built are generally more robust and with a higher prediction accuracy over models built based on GWAS summary statistics. This robustness in comparison to models built on GWAS summary statistics comes from the fact that no additional information (e.g., linkage-disequilibrium info) or assumptions (e.g., shrinkage of the beta estimates) needs to be made to fit these models and all of the data and underlying relationships between features are directly represented in the individual level datasets. Despite the additional computational intensity, using individual level data is a better approach than using summary statistics.

### Platt scaling

In some embodiments, models may be recalibrated as part of the training process. Recalibration may be used to reduce overfitting of each model to its training dataset. This may be advantageous in embodiments where a model is being trained based on data for one population (e.g, European), but will be used in production to provide PRS for a different population. To recalibrate a PRS model, the cumulative effect size of the PRS may be re-estimated using a procedure known as Platt scaling. Briefly, PRS values are calculated for each participant in all datasets. These original values are then standardized to fit the normal distribution. Then, separately in each test set, a secondary generalized linear model may be fit to re-predict the outcome variable using the normalized PRS as a single predictor. These linear models are then used to adjust PRS scores for each individual. As these linear models are trained separately in each dataset, the coefficient of the PRS and the intercept in these models are specific to that dataset, accomplishing recalibration. In some cases, the testing datasets may be ancestry-specific or ancestry- and sex-specific.

### 3. Model Selection & Promotion

Each of the trained models may then be assessed using validation sets to determine various performance metrics. Ancestry-specific model performance may be evaluated using one or more of the following metrics (and corresponding plots): 1) area under the receiver operator curve (AUROC), 2) risk stratification, estimated as odds ratios and relative risks for those in the upper segments of the distribution compared to those in the middle of the distribution (40th to 60th percentiles), 3) an estimation of AUROC within each decade of age - to assess age-related biases in model performance - and 4) calibration plots between PGS quantiles after Platt scaling and phenotype prevalences in each ancestry group. One or more of these metrics may be used to select the best performing model for each ancestry validation set. In some implementations the best performing model may be re-trained using the same SNPset and hyperparameters, but trained on individuals in the train and validation set (rather than just the train set). In other implementations the best performing model for a particular ancestry is promoted for use in production to generate a PRS for that ancestry.

The systems and methods described herein can include various predefined criteria that a model should meet before it can be deployed and used for producing user facing reports to replace a previous version of the model. Different criteria can be used for different models and phenotypes. In some examples, the reclassification rate can be used as part of the criteria. A threshold of 1% could be used for the reclassification rate (as compared to the report outcome for a set of users/test set with a previous version of the model). Another predefined criteria can be the percentage of users that would receive a "Not Determined" result with the model. The predefined criteria can also include the beadchip platform and other information like gender, age, ethnicity, etc. In one example, the predefined criteria could be that "the reclassification rate must be below 1%" and "'Not determined' must comprise less than 5% of users genotyped on the v5 platform."

### Incorporating New Features for Training and Modeling

In some implementations, new features can be incorporated into the training process by grouping SNPs in a gene based on functional information. Functional information on SNPs can be obtained by using a bioinformatic pipeline. A gene-specific functional feature can be created for each gene by grouping SNPs based on their functional role. In some examples, a gene-specific Loss-of-function (LoF) feature can be created by grouping SNPs of a gene based on loss of function characteristics. The LoF can be used as features in the PGS models described herein.

In some examples the model building techniques described herein can be used to generate "functional gene" scores, aka gene-specific LoF features. The method can include identifying LoF variants, group LoF variants into genes, and group LoF variants in coding regions of the gene to create gene-specific LoF gene features. For each individual the LoF gene features can be applied to the individual's data to determine if the individual has at least one broken copy of the gene.

In some examples, phenotypes that have significant association with the gene-specific LoF features can be identified. The methods can include performing statistical analyses to find associations between phenotypes and the gene-specific Lof Features to identify phenotypes for which the gene-specific LoF features show a significant association (analogous to the SNP selection processes described herein). In some examples the effect sizes of the gene-specific LoF features in a model can be compared to the features of individual SNPs in the gene to compare performance.

### 4. PRS Reports

### Interpreter Module and Quality Control Measures

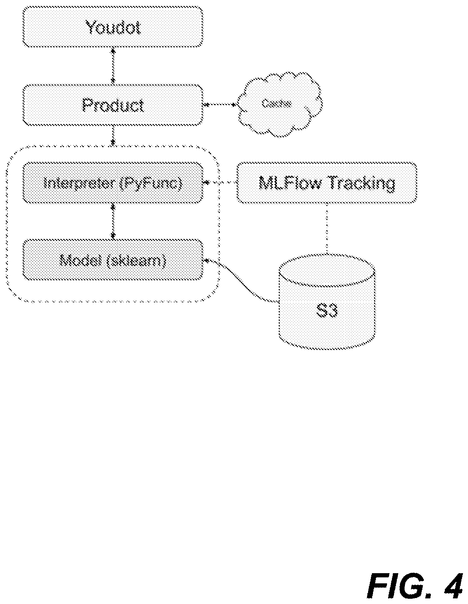

The Interpreter module includes algorithms that can perform a number of features described herein. For example, the Interpreter can perform the Platt scaling, PGS result binarization, estimated likelihoods, and Quality control measures described herein. For example the Interpreter module can generate all of these statistics and save the artifacts required to implement the user-facing content. Figure 4 shows an example of an Interpreter module. When YouDot queries the PRS machine endpoint to get results for a user, the PRS model is applied to their genetic data, and then the interpreter takes over and determines the qualitative/quantitative results and the scale of any uncertainty in the qualitative result (Quality control measures). In some examples a sklearn model can be paired with the Interpreter module or can be part of the Interpreter module. For example a serialed skleam object created during training can be used for prediction.

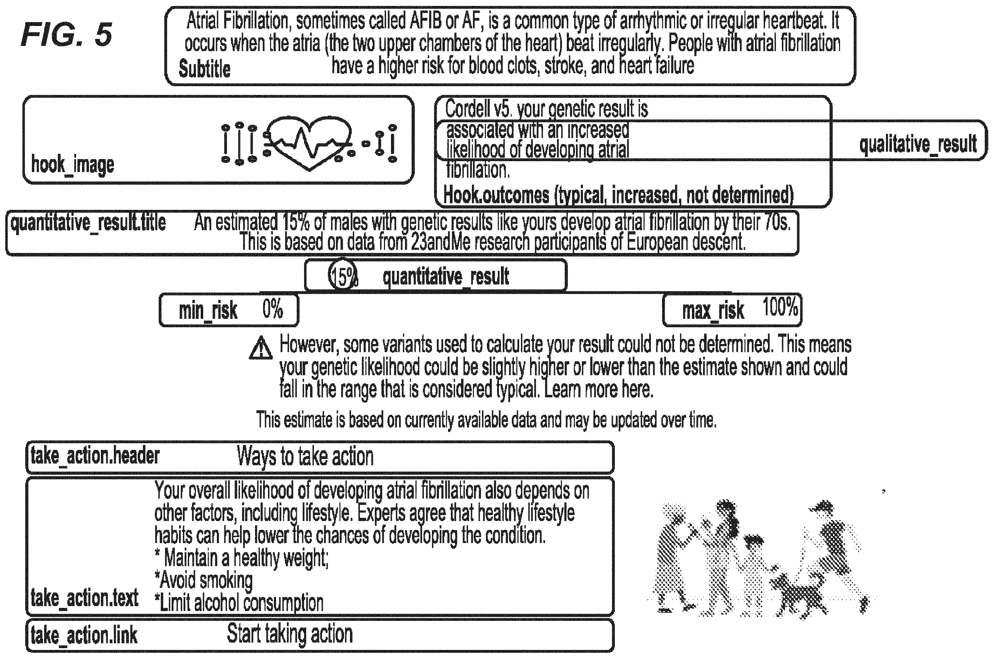

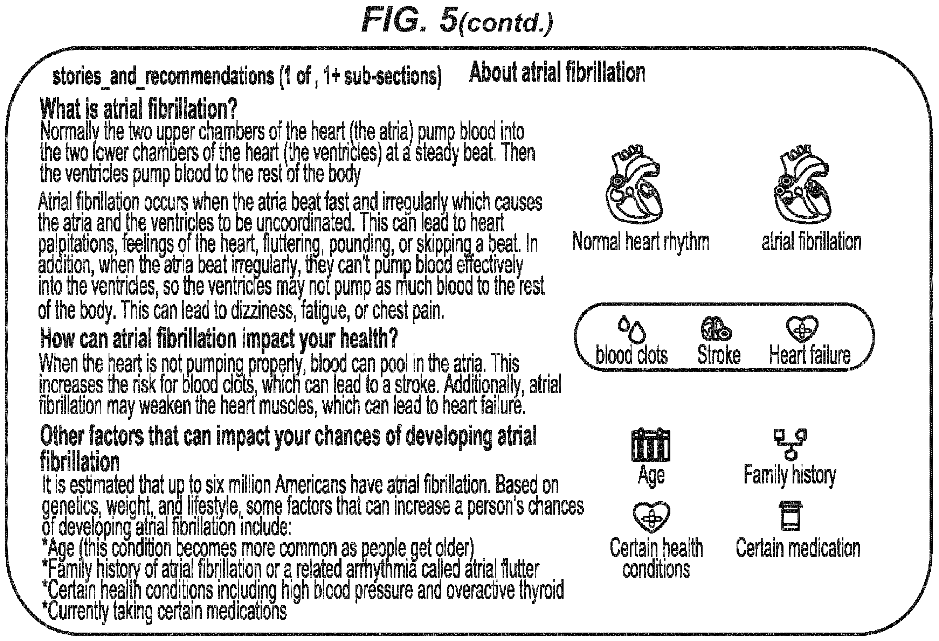

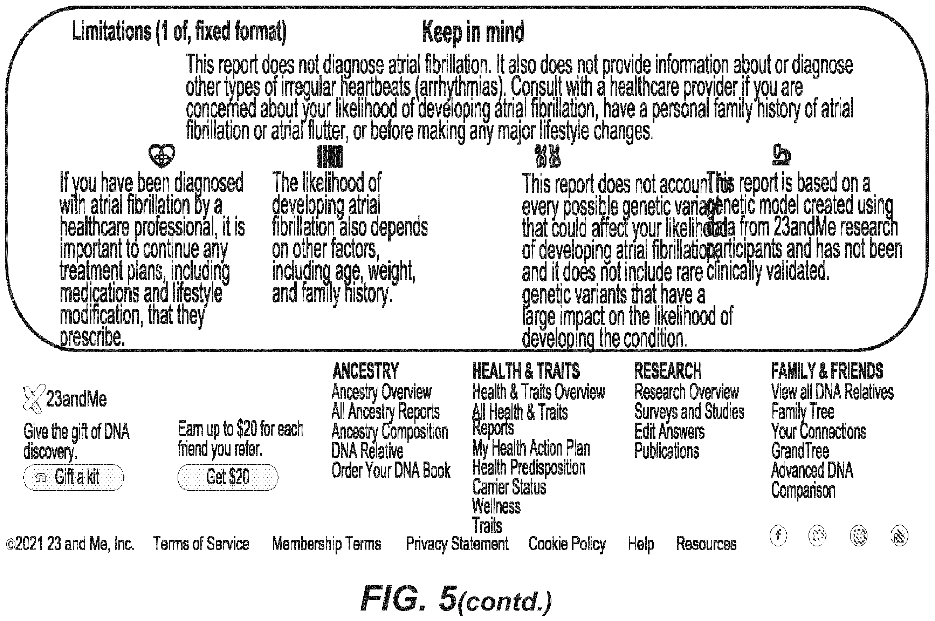

As shown in Figure 4, the product code base points at the interpreter artifact in S3. A youdot query initiates a load of the PRS model and a user's data and then generates the score for the user using the model. The score is then passed through the Interpreter module/algorithms, which returns to Youdot the qualitative and quantitative results. The Interpreter module can perform one or more of Platt scaling, PGS result binarization, estimated likelihoods, and Quality control measures described herein in the process for generating the qualitative and quantitative results based on the user's data. The qualitative and quantitative results can then be used to populate the modular format (see FIG. 5) for the respective report to create the content that is caused to be displayed on the user device.

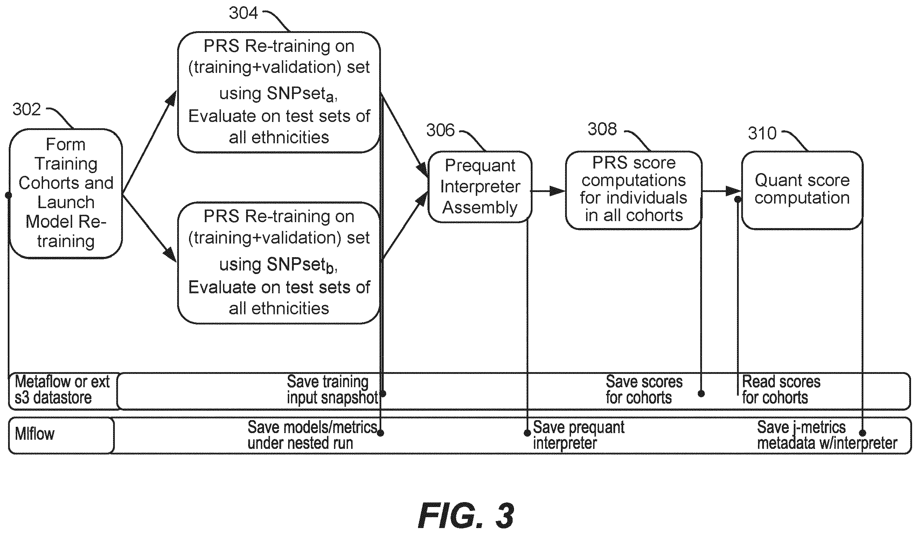

Figure 3 presents a flowchart for using an interpreter module to determine a quantitative result for a user. In block 302 cohorts for training are formed and model training is initiated. In some embodiments block 302 includes one or more of operations 102-14 as described in Figure 1. In block 304, PRS models may be retrained using the training and validation set and then evaluated on tests sets for all ethnicities. In block 306 a prequant interpreter assembly operation is performed to combine the various PRS models. In various embodiments the interpreter module determines which PRS model to use for a particular individual. Thus, the interpreter module may determine all of the PRS models that it may use for generating a report for a phenotype. In block 308 the PRS score is determined for all individuals in a cohort. In some implementations this may be the same individuals in the training cohort.

In block 310 a quantitative score is computed for each individual based on the PRS score and potentially other information. For example, the report result provided for display to a user may indicate a likelihood of developing a condition by a target age. For each customer, the report result may be presented as the likelihood of developing a condition by some target age (e.g. their 70's). This estimated likelihood may be derived by multiplying an estimated genetic relative risk by an age- (and potentially sex- and ancestry-) specific baseline condition prevalence at the target age. Baseline prevalence values may be derived from either external datasets, if available, or the 23andMe database. If there is not a clear match between a population in an externally derived baseline and a 23andMe ancestry group, the European baseline may be provided instead because it is the largest available sample.

In some embodiments, PRS are standardized within each ancestry-specific test set, and PRS distributions are segmented into bins corresponding to percentiles. In some embodiments there may be about 90 or more bins, with the lowest and highest 5% of customers placed into single bins, and 90 intermediate bins each capturing 1% of the PGS distribution between these extremes.

Next, model-estimated prevalences are determined for each genetic result bin at the target age of the report result. In some embodiments this is accomplished by re-estimating the prevalences for the test sets with the age parameter set as the target age (along with age-related covariates like any age-by-sex interaction terms) for the whole test set. In this way, the full (genetics + demographics) model is used to estimate prevalences for each ancestry group at the target age for both sexes. These model-estimated prevalences may be generated because the sample size of every ancestry-specific test set is usually not sufficient to calculate observed prevalences stratified by sex, age, and PRS percentile.

In some embodiments these estimated phenotype prevalences at the target age may be Platt scaled to adjust for any miscalibration within each ancestry group. In some embodiments the parameters used for Platt scaling are based on the distribution of estimated probabilities given participants' actual ages (i.e., Platt scaling parameters are not re-estimated when age is fixed for the whole sample).

These scaled estimated phenotype prevalences are transformed into relative risks with reference to the median of each ancestry group's PGS distribution. In other words, the estimated prevalence for a particular genetic score percentile at the target age for a given sex is divided by the estimated prevalence at the median PGS for that group. The resulting values represent estimated relative risks based on the full model (including both genetic and demographic features) across the dimensions of genetic risk and demographics. These relative risks may then be multiplied by the baseline prevalence values to yield target age-linked estimated likelihoods.

In some embodiments, PRS results are binarized into two categories: one representing individuals at increased likelihood of developing the condition and the other representing typical - i.e., not increased - likelihood of developing the condition. This may be accomplished by determining a threshold (a specific level of risk defined by an odds ratio or relative risk) and then calculating the specific PGS number that corresponds to that threshold such that everyone with a higher PGS has at least that level of risk.

In some embodiments the PRS results are calculated in batches of multiple individuals. In some embodiments the PRS results are calculated on demand when a customer logs in to the 23andMe website.

Quality control measures may perform any one or more of different analyses on the user's data and features of the model. Users will have different SNPs depending on the SNPs that were included in the genotype chip/array/beadchip that was used to generate their genotype. In addition, through the assaying process SNPs that are included on the chip may not yield a definitive result, not be able to be read/determined, or have a high no call rate. In one example, a predetermined threshold for the number of missing SNPs in the user's data that are features in the respective model can be used to determine if a result (e.g. typical/increased risk) or if no result should be provided to the user. In some cases, a threshold of greater than 5% or 10% missing SNPs in the model can trigger providing no result to the user. In another example a weighted combination of SNPs and their respective weights in the model can be used to trigger providing no result to the user. The weighted combination can be further compared to the binarization threshold in some cases.

In yet another example, for phenotype scores generated using imputed user data, the contribution of imputed SNPs to the user's risk score can be evaluated and compared to the user's score and the difference between the user's score and the binarization threshold. If the contribution of the imputed SNPs makes the user's score close to the binarization threshold then providing no result could also be triggered.

In certain embodiments, quality control is conducted using one or more of the following operations, in any combination.
a. Retrieving a PRS model from a database based on the customer data, wherein the PRS model includes a plurality of features including a plurality of genetic variants/SNPs;
   i. Wherein the customer data used for selecting the PRS model comprises one or more of: customer gender and customer genotyping chip version.
b. Retrieving the customer data corresponding to the plurality of features;
c. Comparing the genetic variants/SNPs in the PRS model to the customer data to determine a quantity of genetic variants in the PRS model that are absent from the customer data;
d. Determining if the quantity of genetic variants in the PRS model that are absent from the customer data exceeds about 10% of the genetic variants in the PRS model;
e. Outputting a null result for the PRS model to the customer if the quantity of genetic variants in the PRS model that are absent from the customer data exceeds about 10% of the genetic variants in the PRS model;
f. Calculating a PRS score for the customer based on the PRS model and the customer data corresponding to the plurality of features in the PRS model;
g. Providing a qualitative result to the customer based on whether the PRS score for the customer exceeds a predetermined threshold; and
h. Generate a modular report to cause to be displayed to the customer based on the qualitative result.
i. determining a contribution to the PRS score for the customer based on imputation of genetic variants; comparing the contribution to the PRS based on imputation of genetic variants to the predetermined threshold of the PRS model; and outputting a null result for the PRS model to the customer if the contribution to the PRS based on imputation of genetic variants to the predetermined threshold of the PRS model exceeds a contribution threshold.

In certain embodiments, quality control is implemented by a computational module other than a machine learning model. In certain embodiments, quality control is implemented by an Interpreter module having one or more features as described herein.

In certain embodiments, quality control includes evaluating user genetic data to determine whether the data is missing at least a threshold number of variant allele calls used in a machine learning model under consideration. In some implementations, the threshold number equates to about 10% or greater. In some embodiments, if imputed dosages (variant alleles) are necessary to make the user's predicted phenotype beyond a threshold for increased likelihood of the phenotype, a quality control routine rejects the results, e.g., prevents the results from being displayed to the user.

In some embodiments, the effect of the missing data may be estimated. In order to estimate the uncertainty resulting from missing data, a metric is determined that includes information about a variant's effect size (*β*), its effect allele frequency (p), and an individual's distance from the binary result threshold. For each missing genotype call *i* across *n* missing calls, the below equation may be used to determine the ratio between the distance of an individual's score from the threshold and the uncertainty in the score due to missing values. $\frac{threshold - PGS}{\sqrt{{\sum }_{i = 1}^{n} 2 {β}_{i}^{2} ⋅ {p}_{i} \left(1-{p}_{i}\right)}}$

As this metric approaches zero, the probability that a customer's score could be on the other side of the threshold increases to a maximum of 50%. In some embodiments, if an individual's score has greater than a 1% chance of being on the other side of the binary threshold due to the specific missingness patterns in their data, the customer is alerted to the possibility that their qualitative result could differ if they were genotyped again and these missing values were called.

The processes described herein can enable rapid calculation of the user's risk score, interpretation of the score with the Interpreter module, and preparation of the content for the respective report, such that this process can be calculated on demand when the user logs in to their account or when the user requests to view a specific report. For example, the process of generating the report can be done in less than 1 second.

Generating reports on login/user request for a specific report is one of multiple applications of the model and system. Other examples for steps after generating the reports include triggering a notification to take some action, including the model/report outcome in a downstream prs, phenotype or GWAS studies. Other examples include using the prs outcome for eligibility for a clinical trial, therapy, or reimbursement.

The interpreter module can also select the appropriate version of a particular PRS model based on one or more predicates. Examples of predicates include the genotyping chip version that the user used, such as V1, V2, V3, V4, and V5, gender, etc. For example: a breast cancer model might be valid for "sex=Female and genotyping_chip_version=v5". There can also be different models based on gender and genotyping chip version. A particular version of a mode could be tailored to the SNPs in a particular genotyping chip version.

The interpreter module can also be used to interpret user results, such as multiple genotyping results of the user (genotyped on multiple chip versions or two sets of results on a specific chip version, etc.). High-dimensional genotyping assays include some degree of error and uncertainty. The interpreter is tuned to minimize the "reclassification rate." If a user is genotyped twice, the model is optimized to consider both genotyping results and to minimize the rate that they would receive conflicting results (ie. "elevated risk" vs "typical risk").

### Modular Report templates

Using modular report templates can streamline content generation for reports as well as decrease the response time for calculating a PRS score for a user, converting the score to the report information, and displaying the report to the user. An example of a modular report for Atrial Fibrillation is shown in Figure 5. The modular report design and creation can pull from curated content as well as personalized results from the user's genetic and other data that are input into the model.

Examples of content categories shown in the Atrial Fibrillation modular report illustrated in FIG. 5 include:
Title: report title
Subtitle: additional information on the report
Personalized report result selected from options including: typical risk, increased risk, not determined
Quantitative result on the risk, minimum risk, maximum risk
Ways to take action, additional disease information, limitations to keep in mind,
The quantitative and qualitative results can be received from an interpreter module and populated in a modular report.

The models described herein can be used to predict a variety of different phenotypes. Examples include risk of disease onset, biomarkers like weight, morphology like eye-color , personality traits, etc. Examples of target phenotypes and corresponding modular reports include: type-2 diabetes (T2D), LDL cholesterol, high blood pressure (HBP), coronary artery disease (CAD), atrial fibrillation (Afib), migraine, osteoporosis, insomnia, restless leg syndrome, sleep apnea, sleep quality, sleep need, sleep paralysis, snoring, poly cystic ovary syndrome (PCOS), uterine fibroids, gestational diabetes, endometriosis, morning sickness, age at menopause, preeclampsia, postpartum depression (PPD), non-alcoholic steatohepatitis (NASH), non-alcoholic fatty liver disease (NAFLD), sprint vs distance running, ACL tear likelihood, concussion, elbow tendonitis, bone fracture, herniated disc, joint dislocation, meniscus tear, plantar fasciitis, rotator cuff tear, runner's knee, shin splints, agility, athleticism, balance, court vision, dancing ability, endurance, flexibility, foot-eye coordination, grit, hand-eye coordination, jumping, sprinting, gout, kidney stones, irritable bowel disease, lupus, psoriasis, genetic weight, BMI, triglycerides, cat allergy, dog allergy, etc. The reports can include lifetime risks such as normal or increased likelihood. In some aspects a quantitative result can be provided for a numerical estimate of a phenotype or a numerical estimate of risk. Structure of a PRS Model and packaging

In some implementations A "PRS Model" is composed of two distinct models, one is a standard machine learning model such as a linear/logistic implemented in scikit-leam and the other provides interpretation of model results for consumption in reports. The linear/logistic "model" may be implemented separate from the "interpreter." By separating concerns like this, PRS Machine can (re)train and publish a serialized regression while maintaining the interpreter model, and vice-versa. This allows for experimentation and better debugging of models.

### 5. Continual Model Performance Assessment (Validation)

This disclosure also relates to the monitoring of model performance over time. In some embodiments, when a model is initially deployed, there may be initial performance metrics associated with it based on testing with a test cohort. Over time additional users' genotypes are sequenced and additional phenotype information becomes available that may be used to evaluate a model's performance. For example, users may answer additional survey information that updates their phenotype information. As discussed elsewhere herein, a user's phenotype information may also be inferred based on various information provided by a user. This may result in an updated dataset that would provide different performance metrics for the model being used in production.

In these embodiments, a model may be retested to determine second performance metrics. The particular metrics used may be the same as, or different than, the initial performance metrics. In some embodiments, the second performance metrics are compared against the initial performance metrics to determine a difference. If the difference exceeds a threshold, then a new model may be trained as described elsewhere herein.

In some embodiments, a time threshold is used to determine whether to deploy a new model. For example, if a minimum amount of time has lapsed since the initial model was deployed, a new model may be trained and potentially deployed if it's performance metrics exceed the deployed model's performance metrics. In some embodiments, the time threshold may be weekly, bi-weekly, monthly, quarterly, or yearly. In some embodiments, rather than retraining, a model may be tested to determine additional performance metrics according to the time thresholds above. The additional performance metrics may be compared against one or more of the prior performance metrics, and if the difference between the additional performance metrics and the prior performance metrics exceeds a threshold, a new model may be trained.

In some embodiments, when an updated model is trained and deployed, a notification may be sent to users who have viewed a report based on a PRS from the prior model or who would use the updated model to generate a report.

In addition to the non-technical validation which could include academic collaborations, regular checks against recent publications, a model lifecycle validation process is suggested below.

### Monitoring of model performance on Portal and Email.

Alerts for performance degradation below specified thresholds can trigger a notification and eventually possibly a retrain of the model. Examples of automated performance metric reports that can be generated included distributions of raw data and deviations, AUC and confidence intervals on AUC, and changes from the test set AUC for the served interpreter, etc.

### Scheduled retraining and testing - bi-weekly/monthly/quarterly/yearly

a. Defined train and test sets - congruency of data sets in R&D and production - check for equivalency (external and internal data sets - UK Biobank, others)
b. If retrains pass tests, model version may be updated
c. Email alerts after each retrain with metrics and checks and specified for all PRS models

### 6. Updating a Model

As noted herein a model may be evaluated for determining whether it is still the best model. In some embodiments, a new model may be deployed based on a determination that a model should be updated.

Each model, including models used in production as well as trained models that were not selected/no longer used in production, may be associated with various metadata, where the metadata may include: Model parameters comprising number of SNPs, SNP selection parameters; model metrics (AUCs (genetics and full; full can include genetics and any covariates like age/sex/other demographics), R-squared, Relative risk (top vs. bottom and top vs. middle) Observed Absolute risk (phenotype) diff (top vs. bottom, top vs. middle)), training phenotype, and additional metadata for cohort definition, cohort assembly time, acceptance criteria, validation, and Model specification. All metadata associated with a model may be saved in a repository, allowing for the reproduction of the model, including the training process, using the metadata. On an on-going basis, a researcher may be able to define a model and a PRS Machine that fully supports an end-to-end workflow for (re)training, validation, and deployment in the production environment. Models may be defined in a git repository, trained on production data, and made available in a performant and scalable web service in the "live" production environment.

In some embodiments, it may be determined that a new model should be trained. This may be based on performance metrics for the current model falling below a threshold. In some embodiments, a difference in current vs. historical performance metrics for the current model, as a result of additional data for testing, may exceed a threshold and prompt training a new model. A new model may be trained as described herein by, generally including defining a training, validation, and testing cohort, determining a plurality of SNPsets, and training one or more models based on each SNPset. The result may be a new trained model that has updated metadata associated with it.

In some embodiments, the performance metrics of the new model and the current model may be compared. If the new model has better performance metrics, it may replace the current model. In some embodiments, the new model may not have better performance metrics, and in such cases the current model may remain in production.

In some embodiments the user can be sent an electronic notification informing them that the model has been updated and that their report outcome may have changed. The notification may include an explanation as to why the report outcome may have changed. The updated report can include version tracking such as which version of the report they are viewing (e.g. version 1.0, version 2.0) along with the corresponding release date for the respective version that they are viewing.

### Reproducibility

Given a unique set of high-level parameters defining a PRS Machine model, i.e. metadata associated with a model, the PRS Machine should be able to train and deploy a model with reasonable guarantees that subsequent attempts at retraining and deploying a model produce an acceptable model for use in the 23andMe consumer product. In the unlikely event of a catastrophic failure in which the trained models become unrecoverable, PRS Machine should be able to rebuild and redeploy those same models with the same guarantees provided in the original release cycle.

Reproducibility is a desirable trait for offline investigation and debugging. A system that supports offline debugging and reproducibility ensures that production issues may be investigated and potentially fixed with minimal disruption to live systems.

### Model Training Specification

As noted above, a PRS Machine repository may contain shared code for interpretation of model results, preprocessing, or other non-inference-related activity. A specification file or other form of storing parameter information may be used to provide parameters for each part of the PRS model training process. This specification file may be stored and tracked to allow for updating a model and maintaining the parameters used for training current models in production.

A major benefit of tracking the parameters for training a PRS model is that the training process may be performed and reproduced without intervention by an engineer or data scientist during the training. Models may be trained by specifying all of the parameters in a specification file that is then executed by a PRS machine without further user input, rather than requiring manual decisions by a data scientist at various points, for example to split datasets into train, validate, and test sets. The parameters act as rules for the training process that may be configured by a data scientist without having to manually perform various operations, such as loading user data into a cache for parallelized training.

In some implementations, changes to code and/or references in the prs-machine repository may trigger the same CI and deployment pipeline as if a model were published.

As noted above, in some implementations a PRS model may be defined by a set of parameters that define rules for performing various parts of the timeline. In some embodiments, the parameters may include one or more of the following:
[1] SNPs curated from literature / known associations;
[2] Target phenotype (e.g. T2D, etc.);
[3] Previously run GWAS jobs to use for the analysis;
[4] test set threshold - number of cases to form validation/test sets;
[5] validation set threshold- required number of cases to form training/validation/test sets. In some embodiments the test threshold is 4,000 cases to form validation/test sets. In some embodiments the validation threshold is 8,000 cases to form train/validation/test sets;
[6] validation / test set ratio (0, 5, 5);
[7] training validation, and test set split (7/2/1 - 8/1/1);
[8] imputation panel;
[9] GWAS covariates - sex, age, beadchip platform (V3, V4, V5), principal components (European, All);
[10] Minimum number of SNPs, maximum number of SNPs;
[11] P-values - 1, 0.5, 0.1, 0.01, 0.001, 0.0001, 0.00001, 0.000001, 0.0000001, 0.00000001 and lower, other arbitrary ranges can be specified;
[12] P-value and/or distance pruning - choose selection;
[13] SNP Windows (physical distance between base pairs) 0, 10,000, 50,000, 100,000, and 500,000;
[14] Miscellaneous: specific 23andMe report ID, model name;
[15] Phenotypic features to include in the model training, in addition to the genetic components: age, gender, BMI, etc;
[16] Principal components;
[17] Age (min / max);
[18] Sex/gender filter;
[19] Model solver (logistic regression types of solvers: skleam, lbfgs, etc.) and settings for solver (model penalty, max iterations), specify prediction formula format, qualitative/quantitative results, bins for model results, etc.;
[20] Model selection criteria: AUC, others;
[21] Population specific ethnicities: African America, East Asian, European, Latino, South Asian, West Asian, etc.;
[22] Baseline, for example baseline prevalence of the phenotype of interest for each ethnicity;
[23] Distance pruning;
[24] Allowlist - curated selection of SNPs from the beadchip that have passed QC metrics. Typically a microarray or beadchip can test for on the order of 1,000,000 to 1,500,000 SNPs. QC metrics and other filtering can be done on the SNPs to create a curated list of SNPs for model building. For example, in some cases the allow list of SNPs can be on the order of about 300,000 to about 400,000 SNPs;
[25] GWAS sample sizes.

### Privacy, Security, and Compliance Features

Certain methods described herein build in privacy and compliance considerations. For example, the methods can ensure privacy as well as compliance with various laws and standards (e.g., ISO27001, GDPR, CCPA, IRB compliant, HIPAA, etc.).

Privacy laws in some jurisdictions, such as CCPA and GDPR, may require personal data to be deleted within a certain time frame of receiving a deletion request from a user. In some implementations, when a deletion request is received from a customer, all personal data are deleted from the upstream source databases. Lifecycle policies may be defined in the PRS machine to delete all temporary caches of personal data within, e.g., 30 days of storage or use to ensure GDPR / CCPA compliance.

In some embodiments, all training runs start with currently consented data. Temporary caches are used in some of the steps described herein. For example, the parallel machine learning training of models can cache individual level data. The preparation of a GWAS can also cache individual level data. GDPR and CCPA compliance can be achieved by deleting any cached individual data within, e.g., 30 days of saving it to a cache. In some cases, any new model training or GWAS will only include individual level data that is consented for those uses.

Part of IRB and other compliance regimens includes using only data corresponding to customers who have consented to their data being used for research. IRB and other consent agreements, geographic locale, and other attributes relevant to consent are available to be used in predicates when defining inclusion criteria for the training steps (GWAS and regression training). Participants may withdraw their consent at any time and future training runs respect those preferences.

Security measures may be included in the methods and systems described herein. Roles may be separated between software development, system deployment, system maintenance, and model authorship. Using the 'model author' role, models may be authored, automated acceptance criteria defined, and performance statistics of the models may be viewed without access to the highly sensitive individual-level customer data or elevated access to the running PRS machine system. As such, "model authorship" can be extended to a broad set of individuals including non-employees. All queries for model inference may be encrypted & logged in accordance with, e.g., HIPAA & ISO27001 security frameworks and access may be tightly controlled.

In some embodiments various privacy protections are built into the PRS pipeline. Privacy may be preserved by deleting individual level data under certain circumstances. For example, GDPR delete requests, CCPA delete requests, or delete requests made pursuant to other privacy rules or regulations may require removing individual level data from a database that is used to build PRS models. The described embodiments may comply with the requirements of GDPR, CCPA, and/or other privacy rules or regulations.

In some embodiments, the process of developing a machine learning model is characterized by any one or more of the following procedures:
[1] Storing genetic data and phenotypic information for a plurality of customers who have provided consent to allow their data to be used for research through a user interface;
[2] Separating the genetic data and phenotypic information for the plurality of customers into a set of cases and a set of controls for a GWAS;
[3] Running the GWAS on the set of cases and a set of controls to generate a statistical dataset of genetic associations for a phenotype of interest;
[4] Storing the statistical dataset of genetic associations for the phenotype of interest and individual level data for a subset of the plurality of customers in a temporary cache;
[5] Running in parallel a plurality of machine learning processes on the statistical dataset of genetic associations and the individual level data for the subset of the plurality of customers to generate a plurality of trained models; and
[6] Deleting the temporary cache of individual level data for the subset of the plurality of customers in the temporary cache within 30 days of storing the individual level data in the temporary cache;
[7] In some cases, a customer's individual level data is deleted in response to the individual making a request to delete his or her data. The deletion may occur before deleting the temporary cache of individual level data.

In certain embodiments, the customers are customers of a personal genetics service such as 23andMe's personal genetics service. In certain embodiments, the personal genetics service interfaces with customers via a computer user interface, such as a web-based user interface. In certain embodiments, the user interface is configured to receive customer consent to participate in research and/or customer delete requests for deleting individual level information.

In certain embodiments, the subset of the plurality of customers is a subset of all or many of the customers who have consented to allow their data to be used for research. In some cases, the subset of customers is limited to customers selected to be used in research leading to developing one or more machine learning models for predicting a designated phenotype from genetic information. In some cases, the subset of customers is limited to customers having individual level information selected for use in performing a GWAS and/or in generating the one or more machine learning models for predicting a phenotype from genetic information.

Individuals may consent in various ways to having their phenotype information and/or genetic data used for research. A user may consent to having his or her answers to survey or form-based questions used in the research. A user may consent to having his or her information about health, age, gender, ethnicity, and the like used for research. In some cases, a user provides consent to use his or her information to discover genetic factors behind diseases and traits and/or to uncover connections among diseases and traits. In some cases, consent is qualified to give researchers access to a user's genetic and other personal information, but not to his or her name, contact, or credit card information. The research that a user consents to may include development of computational tools such machine leaming models of the types described herein. A user's consent may also extend to GWASs. In some cases, users consent via inputs to a web browser or other user interface on a computer system. In some cases, the users may provide their consent via a user interface for a personal genetic service such as one that also provides the user with information about one or more predicted phenotypes produced using one or more machine learning models such as any of those described herein.

In some implementations, individual-level information includes at least some of the individual's genetic information and information. It may also include ethnicity, gender, age, and/or other phenotypic characteristics. The phenotype information may include self-reported phenotype information such as physical characteristics (e.g., height, weight, eye color, sensory abilities, etc.), diseases, and other medical conditions.

In certain embodiments, the statistical dataset is a curated list of SNPs and/or other polymorphisms identified as having an impact on a phenotype of interest for a machine learning model and/or a GWAS. In some implementations, the statistical data set is generated by a GWAS using individual level information. In some implementations, the statistical dataset comprises SNP and/or other polymorphisms and associated p-values or other indicia of their relative importance to the phenotype of interest.

In certain embodiments, a temporary cache is used to store individual level information used to conduct a GWAS. In certain embodiments, a temporary cache is used to store individual level information and, optionally, the statistical dataset, for training one or more machine leaming models. In some implementations, a temporary cache is used to store individual level information and, optionally, the statistical dataset, for training a plurality of machine learning models. In some implementations, multiple temporary caches are used to store individual level information and, optionally, the statistical dataset, for training each of a plurality of machine learning models.

In some systems, researchers and/or model developers are given roles having associated security levels. For example, in some implementations, researchers and/or developers associated with generating the models do not have access to individual level information

### Examples

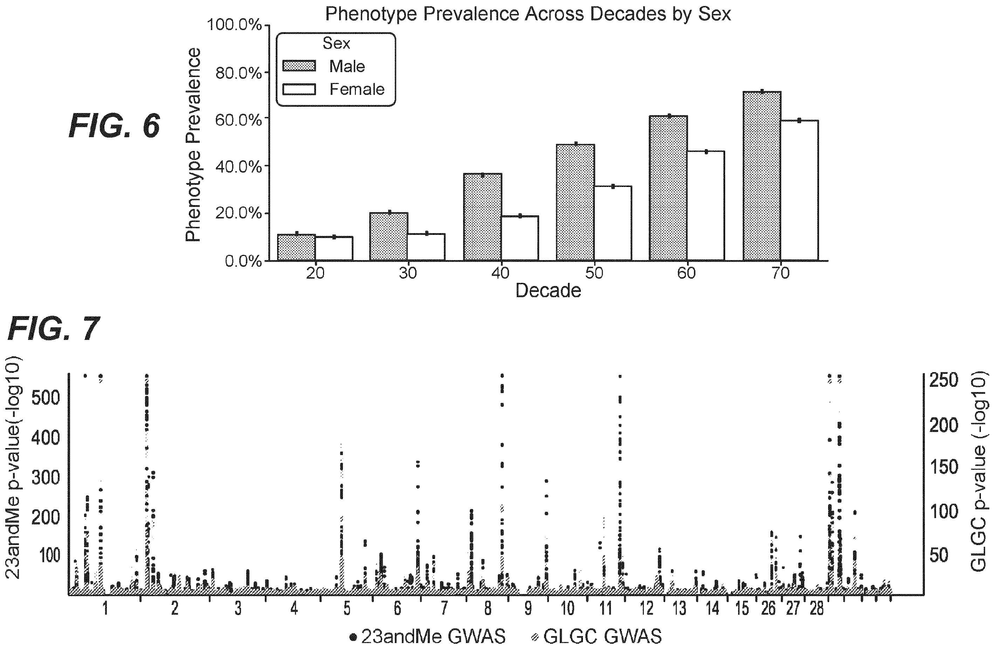

Figures 6-12 relate to an example of determining PRS models for predicting the genetic risk of high LDL cholesterol (LDL-C) levels. Data for the LDL cholesterol model were 23andMe customers who provided informed consent and answered survey questions pertaining to LDL-C cholesterol and a history of cholesterol-lowering medication. Cases and controls were defined in two stages of logic. In the first stage, questions about recent and highest ever LDL-C levels were combined into a single phenotype representing ever having reported LDL-C above 160 mg/dL. Individuals who answered 160 mg/dL or above for either LDL question were counted as cases. Those who answered below 160 mg/dL for both questions were counted as controls, as were participants who answered below 160 md/dL for one question but who did not answer the other. In the second step, prescription medication information was used to infer that a participant ever had high LDL-C. Specifically, among those with self-reported LDL-C lab values data, controls were changed to cases if they indicated a history of being prescribed medication to lower their cholesterol. This step accounts for the fact that, for those individuals, self-reported values may have been concurrent with medical management of LDL-C (i.e., lowered by medication), and thus individuals without high LDL-C at the time of self-report may still have had a history of high LDL-C. Statistics about the cohorts are provided in table 1 below:

**Table 1: High LDL-C participant cohort descriptives**

| **Platform** | **Ancestry Group** | **Sample Use** | **N** | **Age mean (SD)** | **Sex (% female)** | **High LDL-C prevalence (%)** |
|---|---|---|---|---|---|---|
| V1 to V5 | European | GWAS | 617,165 | 56.2 (13.8) | 54.60% | 41.99% |
| V5 | European | Training the European Model | 511,469 | 55.0 (13.9) | 55.50% | 40.60% |
| | | Validation | 115,079 | 54.9 (13.9) | 55.50% | 41.20% |
| | | Testing | 56,749 | 55.1 (14.0) | 55.24% | 40.94% |
| | Sub-Saharan African/African American | Testing | 18,710 | 50.1 (13.5) | 59.02% | 40.94% |
| | East/Southeast Asian | Testing | 18,357 | 44.7 (14.2) | 57.51% | 27.07% |
| | Hispanic/Latino | Testing | 72,806 | 47.8 (14.0) | 56.46% | 33.86% |
| | South Asian | Testing | 6,128 | 44.3 (13.0) | 37.73% | 34.48% |
| | Northern African/Central & Western Asian | Testing | 5,267 | 49.4 (14.7) | 40.38% | 38.47% |

Figure 6 provides survey results for self-reports of ever having had high LDL-C or ever having been prescribed medication to lower cholesterol, an indication that a physician likely determined that the respondent had high LDL-C. This phenotype combined responses from three questions pertaining to the most recent LDL-C, highest ever LDL-C, and medication history. As seen in Figure 6, prevalence increased with advancing age.

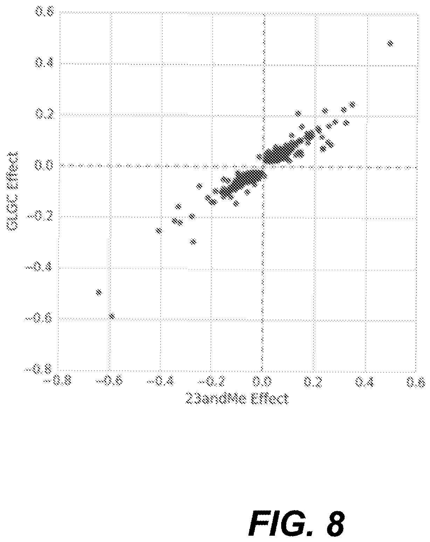

Next, as an additional validation of the 23andMe GWAS, the effect sizes of all independent genome-wide significant loci found in both sets of summary statistics were compared. These effect sizes should be similar in scale and with the same positive or negative valence. The correlation between these two sets of effect sizes was determined after reformatting the data to align all strand and reference alleles and selecting independent variants using clumping and pruning procedures in PLINK (Chang et al., 2015; Purcell et al., 2007; parameters *p*-value = 5e-8, r² = 0.5, distance = 250kb). Figure 7 is a Manhattan plot of 23andMe and Willer GWAS summary statistics for LDL-C. Willer et al., Global Lipids Genetics Consortium. (2013). Discovery and refinement of loci associated with lipid levels. Nature Genetics, 45(11), 1274-1283. https://doi.org/10.1038/ng.2797. Figure 8 is a scatter plot showing the estimated effect sizes for (change in log-odds per unit predictor change) between 23andMe and Global Lipids Genetics Consortium (GLGC; linear betas; Willer et al., 2013) genome-wide significant hits shared between the two GWAS for LDL cholesterol. As shown in Figure 8, all but two genome-wide significant loci showed the same positive or negative valence in the GWAS, and the effect sizes were strongly correlated. The replication of the majority of previously identified loci in addition to the correlated effect sizes demonstrates that the 23andMe GWAS based on self-reported data adequately captured the results of the external GLGC GWAS, which was based on clinically ascertained laboratory values.

### Model performance

Demographic covariates included in polygenic modeling for LDL-C were age, sex, age², as well as sex-by-age and sex-by-age² interaction terms. Model training and hyperparameter tuning was performed in samples of European descent. The final selected model contained 2,950 genetic variants.

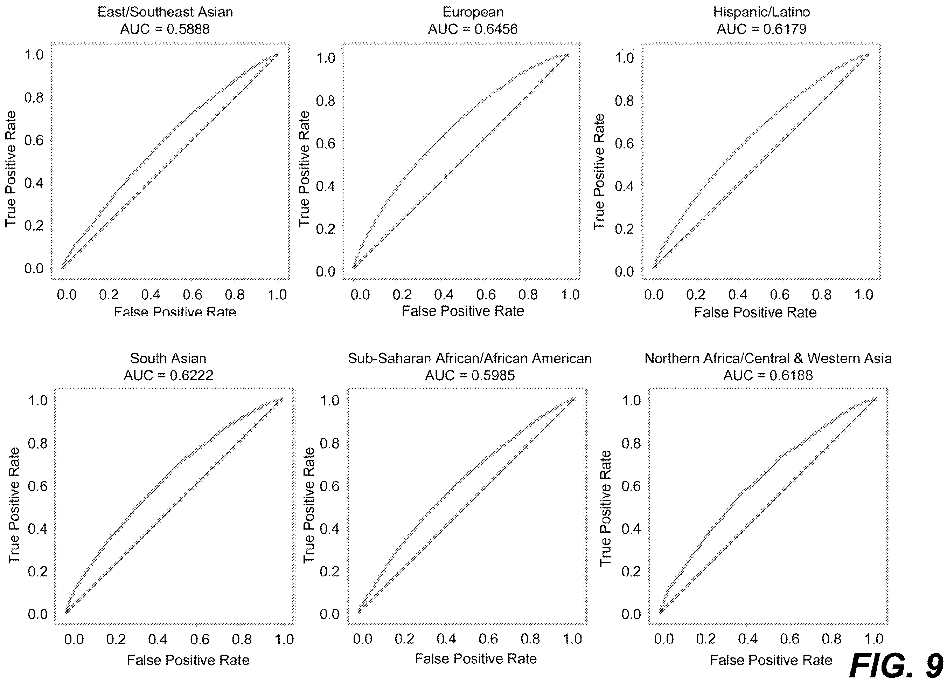

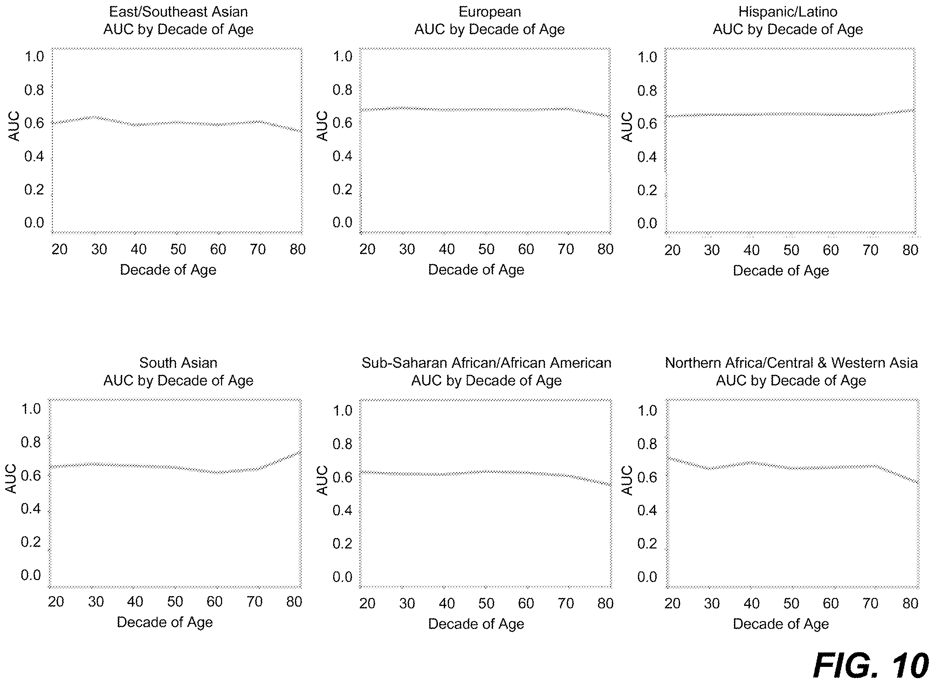

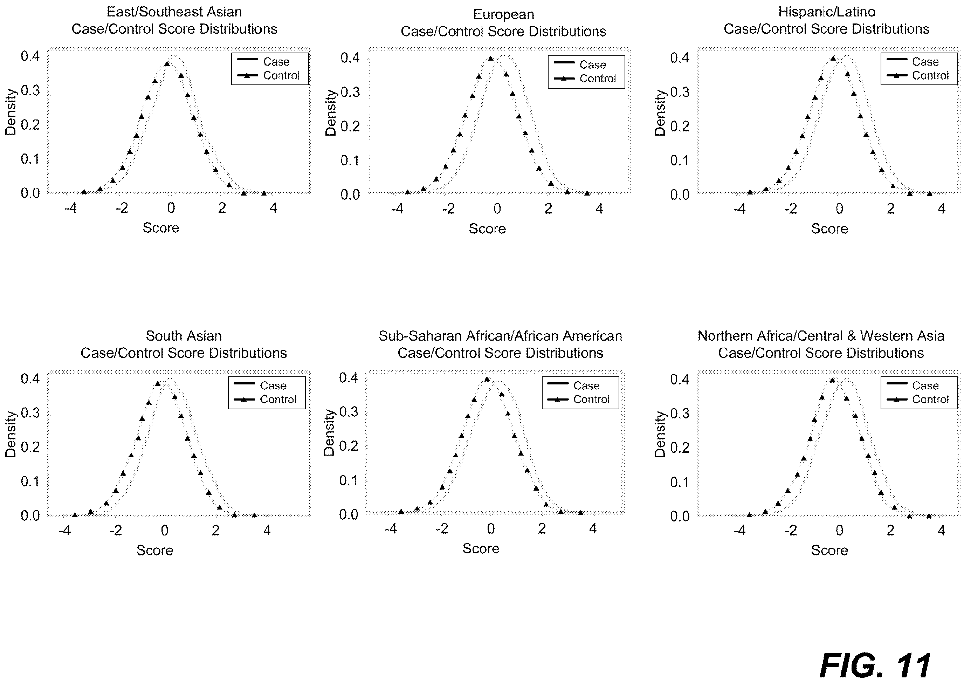

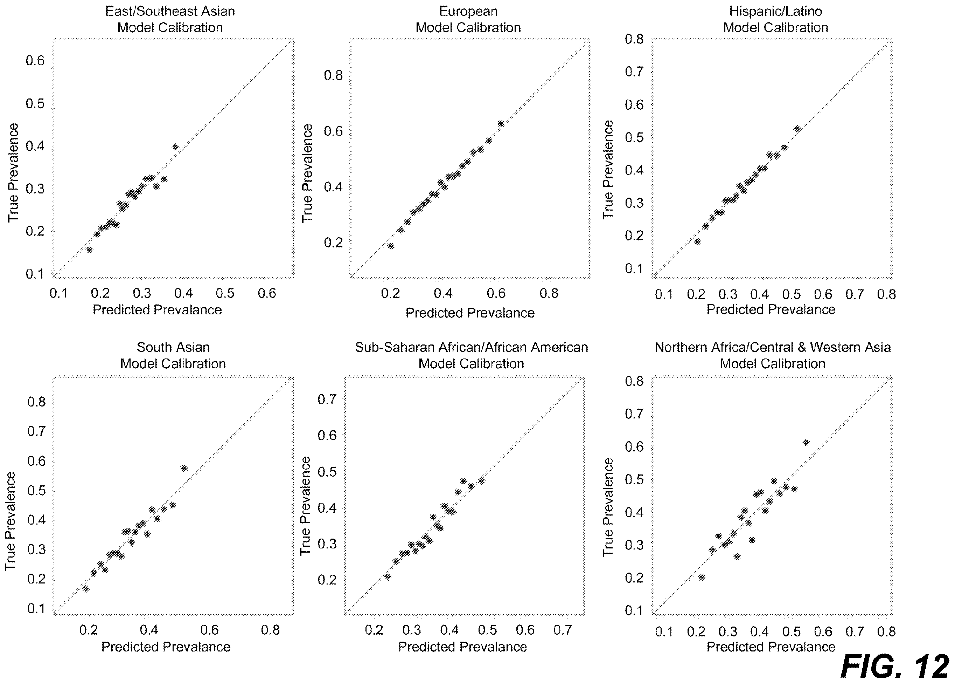

For each of these model-dataset combinations, performance and calibration statistics were assessed. As expected, the PGS performed best in individuals of European ancestry, followed by individuals of Hispanic/Latino, South Asian, and Northern African/Central & Western Asian ancestry, and finally in Sub-Saharan African/African American and East/Southeast Asian ancestries (Table 2, Figures 9-12). Figure 9 shows high LDL-C area under the receiver operator curve (AUROC) across ancestry-specific test sets. Figure 10 shows high LDL-C AUROC within each decade of age across ancestry-specific test sets. Figure 11 shows high LDL-C case/control standardized PGS distributions across ancestry-specific test sets. Figure 12 shows high LDL-C Platt-scaled calibration plots across ancestry-specific test sets. In all these populations, the odds ratio for high LDL-C for individuals in the top 5% of the (genetics-only) PGS versus individuals with average PGS was close to or higher than two, indicating that the PGS was able to stratify a substantial amount of risk for those at the right tail of the distribution. Additionally, calibration plots illustrate a high correlation of predicted versus real prevalence in all ancestries (Figure 8).

### Qualitative result thresholding

We used standardized (within each population) polygenic scores to determine the population-specific threshold corresponding to an odds ratio of 1.5 relative to the 40th to 60th percentile of each population's distribution. Table 3 shows the proportion of customers above this threshold, who would thus receive the "increased likelihood" result. Likelihood ratios associated with the "increased likelihood" result are also provided in Table 3.

**Table 2: High LDL-C PGS performance characteristics**

| **Ancestry Group (test sets)** | **Full Model AUROC** | **Genetics Only AUROC** | **Odds Ratio top 5% versus average (95%CIs)** | **Odds Ratio top 5% versus bottom 5% (95%CIs)** |
|---|---|---|---|---|
| European | 0.7770 | 0.6456 | 2.81 (2.58 to 3.07) | 10.24 (9.02 to 11.63) |
| Sub-Saharan African/African American | 0.7312 | 0.5985 | 1.91 (1.67 to 2.23) | 4.10 (3.34 to 5.05) |
| East/Southeast Asian | 0.7635 | 0.5888 | 1.91 (1.64 to 2.22) | 4.30 (3.43 to 5.39) |
| Hispanic/Latino | 0.7561 | 0.6179 | 2.31 (2.15 to 2.49) | 5.87 (5.27 to 6.55) |
| South Asian | 0.7828 | 0.6222 | 2.69 (2.08 to 3.47) | 7.75 (5.29 to 11.37) |
| Northern African/Central & Western Asian | 0.7776 | 0.6188 | 2.81 (2.13 to 3.72) | 7.49 (5.04 to 11.14) |

**Table 3: High LDL-C qualitative result characteristics**

| **Ancestry Group (test sets)** | **Odds Ratio for Result Threshold** | **Percent Above Threshold** | **Likelihood Ratio of "Increased" Result (95% CIs)** |
|---|---|---|---|
| European | 1.5 | 22.79% | 1.97 (1.91 to 2.03) |
| Sub-Saharan African/African American | 1.5 | 12.32% | 1.69 (1.56 to 1.82) |
| East/Southeast Asian | 1.5 | 10.37% | 1.63 (1.50 to 1.78) |
| Hispanic/Latino | 1.5 | 17.19% | 1.80 (1.74 to 1.86) |
| South Asian | 1.5 | 18.29% | 1.82 (1.64 to 2.02) |
| Northern African/Central & Western Asian | 1.5 | 17.47% | 1.85 (1.64 to 2.08) |

### Quantitative result calculation

Ancestry- and sex-specific baseline prevalences of ever having had high LDL cholesterol were derived from the 2017 data release of the Behavioral Risk Factor Surveillance System (BRFSS; Centers for Disease Control and Prevention [CDC], 2017). The specific calculated variable (coded _RFCHOL1) represents the concept: adults who have had their cholesterol checked and have been told by a doctor, nurse, or other health professional that it was high. The ancestry variable used (coded_RACE) included the categories White only non-Hispanic, Black only non-Hispanic, Asian only non-Hispanic, and Hispanic. Analysis was restricted only to those between the ages of 70 and 79, to capture this decade of age (coded _AGEG5YR). The descriptives used for each sex and ancestry combination and how they map to each 23andMe ancestry group are shown in Table 4.

**Table 4: High LDL-C baseline prevalences**

| **Group** | **Matched 23andMe Population(s)** | **Sex** | **N** | **Prevalence** | **95% CI** |
|---|---|---|---|---|---|
| White Non-Hispanic | European, Northern African/Central & Western Asian, Other | Male | 23,256 | 55.02% | 54.38% to 55.66% |
| | | Female | 33,369 | 54.22% | 53.69% to 54.76% |
| Black Non-Hispanic | Sub-Saharan African/African American | Male | 1,335 | 52.58% | 49.91% to 55.26% |
| | | Female | 2,795 | 53.42% | 51.57% to 55.27% |
| Asian Non-Hispanic | East/Southeast Asian, South Asian | Male | 327 | 46.18% | 40.77% to 51.58% |
| | | Female | 386 | 51.30% | 46.31% to 56.28% |
| Hispanic | Hispanic/Latino | Male | 951 | 46.58% | 43.41% to 49.75% |
| | | Female | 1,619 | 51.95% | 49.51% to 54.38% |

### Computational Embodiments

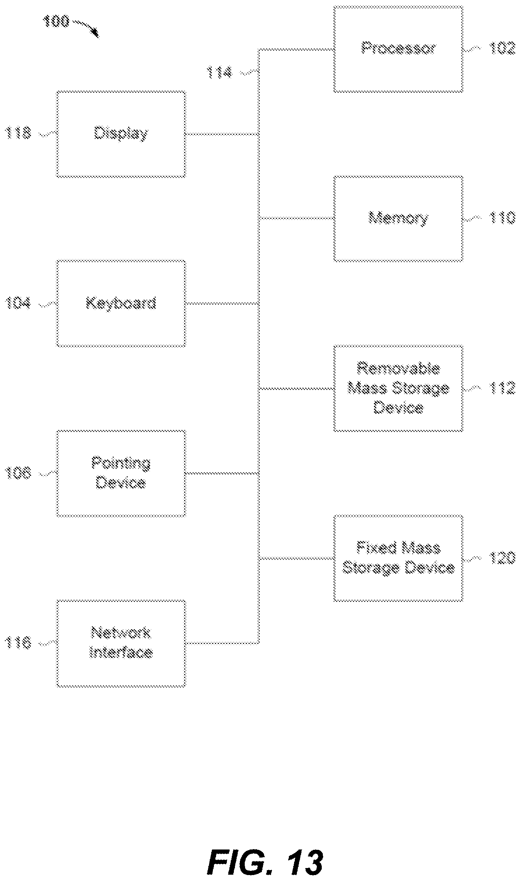

Figure 13 is a functional diagram illustrating a programmed computer system for making phenotype predictions in accordance with some embodiments. As will be apparent, other computer system architectures and configurations can be used to perform phenotype predictions. Computer system 100, which includes various subsystems as described below, includes at least one microprocessor subsystem (also referred to as a processor or a central processing unit (CPU)) 102. For example, processor 102 can be implemented by a single-chip processor or by multiple processors. In some embodiments, processor 102 is a general purpose digital processor that controls the operation of the computer system 100. Using instructions retrieved from memory 110, the processor 102 controls the reception and manipulation of input data, and the output and display of data on output devices (e.g., display 118). In some embodiments, processor 102 includes and/or is used to implement the flowchart of Figure 1.

Processor 102 is coupled bi-directionally with memory 110, which can include a first primary storage, typically a random access memory (RAM), and a second primary storage area, typically a read-only memory (ROM). As is well known in the art, primary storage can be used as a general storage area and as scratch-pad memory, and can also be used to store input data and processed data. Primary storage can also store programming instructions and data, in the form of data objects and text objects, in addition to other data and instructions for processes operating on processor 102. Also as is well known in the art, primary storage typically includes basic operating instructions, program code, data, and objects used by the processor 102 to perform its functions (e.g., programmed instructions). For example, memory 110 can include any suitable computer readable storage media, described below, depending on whether, for example, data access needs to be bi-directional or uni-directional. For example, processor 102 can also directly and very rapidly retrieve and store frequently needed data in a cache memory (not shown).

A removable mass storage device 112 provides additional data storage capacity for the computer system 100, and is coupled either bi-directionally (read/write) or uni-directionally (read only) to processor 102. For example, storage 112 can also include computer readable media such as magnetic tape, flash memory, PC-CARDS, portable mass storage devices, holographic storage devices, and other storage devices. A fixed mass storage device 120 can also, for example, provide additional data storage capacity. The most common example of mass storage 120 is a hard disk drive. Mass storage 112 and 120 generally store additional programming instructions, data, and the like that typically are not in active use by the processor 102. It will be appreciated that the information retained within mass storage 112 and 120 can be incorporated, if needed, in standard fashion as part of memory 110 (e.g., RAM) as virtual memory.

In addition to providing processor 102 access to storage subsystems, bus 114 can be used to provide access to other subsystems and devices. As shown, these can include a display monitor 118, a network interface 116, a keyboard 104, and a pointing device 106, as well as an auxiliary input/output device interface, a sound card, speakers, and other subsystems as needed. For example, the pointing device 106 can be a mouse, stylus, track ball, or tablet, and is useful for interacting with a graphical user interface.

The network interface 116 allows processor 102 to be coupled to another computer, computer network, or telecommunications network using a network connection as shown. For example, through the network interface 116, the processor 102 can receive information (e.g., data objects or program instructions) from another network or output information to another network in the course of performing method/process steps. Information, often represented as a sequence of instructions to be executed on a processor, can be received from and outputted to another network. An interface card or similar device and appropriate software implemented by (e.g., executed/performed on) processor 102 can be used to connect the computer system 100 to an external network and transfer data according to standard protocols. For example, various process embodiments disclosed herein can be executed on processor 102, or can be performed across a network such as the Internet, intranet networks, or local area networks, in conjunction with a remote processor that shares a portion of the processing. Additional mass storage devices (not shown) can also be connected to processor 102 through network interface 116.

An auxiliary I/O device interface (not shown) can be used in conjunction with computer system 100. The auxiliary I/O device interface can include general and customized interfaces that allow the processor 102 to send and, more typically, receive data from other devices such as microphones, touch-sensitive displays, transducer card readers, tape readers, voice or handwriting recognizers, biometrics readers, cameras, portable mass storage devices, and other computers.

In addition, various embodiments disclosed herein further relate to computer storage products with a computer readable medium that includes program code for performing various computer-implemented operations. The computer readable medium is any data storage device that can store data which can thereafter be read by a computer system. Examples of computer readable media include, but are not limited to, all the media mentioned above: magnetic media such as hard disks, floppy disks, and magnetic tape; optical media such as CD-ROM disks; magneto-optical media such as optical disks; and specially configured hardware devices such as application-specific integrated circuits (ASICs), programmable logic devices (PLDs), and ROM and RAM devices. Examples of program code include both machine code, as produced, for example, by a compiler, or files containing higher level code (e.g., script) that can be executed using an interpreter.

The computer system shown in FIG. 13 is but an example of a computer system suitable for use with the various embodiments disclosed herein. Other computer systems suitable for such use can include additional or fewer subsystems. In addition, bus 114 is illustrative of any interconnection scheme serving to link the subsystems. Other computer architectures having different configurations of subsystems can also be utilized.

### Conclusion

In the description above, for purposes of explanation only, specific nomenclature is set forth to provide a thorough understanding of the present disclosure. However, it will be apparent to one skilled in the art that these specific details are not required to practice the teachings of the present disclosure.

The language used to disclose various embodiments describes, but should not limit, the scope of the claims. For example, in the previous description, for purposes of clarity and conciseness of the description, not all of the numerous components shown in the figures are described. The numerous components are shown in the drawings to provide a person of ordinary skill in the art a thorough, enabling disclosure of the present specification. The operation of many of the components would be understood and apparent to one skilled in the art. Similarly, the reader is to understand that the specific ordering and combination of process actions described is merely illustrative, and the disclosure may be performed using different or additional process actions, or a different combination of process actions.

Each of the additional features and teachings disclosed herein can be utilized separately or in conjunction with other features and teachings for protective coverings. Representative examples using many of these additional features and teachings, both separately and in combination, are described in further detail with reference to the attached drawings. This detailed description is merely intended for illustration purposes to teach a person of skill in the art further details for practicing preferred aspects of the present teachings and is not intended to limit the scope of the claims. Therefore, combinations of features disclosed in the detailed description may not be necessary to practice the teachings in the broadest sense, and are instead taught merely to describe particularly representative examples of the present disclosure. Additionally and obviously, features may be added or subtracted as desired. Accordingly, the disclosure is not to be restricted except in light of the attached claims and their equivalents.

Moreover, the various features of the representative examples and the dependent claims may be combined in ways that are not specifically and explicitly enumerated in order to provide additional useful embodiments of the present teachings. It is also expressly noted that all value ranges or indications of groups of entities disclose every possible intermediate value or intermediate entity for the purpose of original disclosure, as well as for the purpose of restricting the claimed subject matter. It is also expressly noted that the dimensions and the shapes of the components shown in the figures are designed to help to understand how the present teachings are practiced, but not intended to limit the dimensions and the shapes shown in the examples.

## Claims

1. A method for generating a polygenic risk score (PRS) model to predict phenotypes of a user, the method comprising:
receiving user-selected parameters related to a PRS model including a phenotype of interest;
selecting and obtaining genetic data for a plurality of individuals based on the user-selected parameters, the genetic data including data pertaining to a presence or absence of the phenotype of interest in the plurality of individuals;
determining a plurality of population-specific genetic datasets from the genetic data based on the plurality of individuals;
analyzing the population-specific genetic datasets to determine one or more sets of SNPs that are statistically associated with the phenotype of interest for that population-specific genetic dataset, wherein each set of SNPs corresponds with one population-specific genetic dataset;
applying SNP filtering criteria to the one or more sets of SNPs to generate a plurality of training SNP sets, wherein the SNP filtering criteria is based at least in part on the user-selected parameters;
loading into a cache of a computer system genetic and phenotypic information for the plurality of individuals in each population-specific genetic dataset;
training a plurality of models using machine-learning techniques based at least in part on the genetic and phenotypic information for the plurality of individuals in the cache, the plurality of training SNP sets, and the phenotype of interest;
generating one or more performance metrics for each of the plurality of models for each of the population-specific genetic datasets; and
determining a plurality of population-specific models based on the one or more performance metrics.

2. The method of claim 1, further comprising:
determining a combined set of SNPs that are statistically associated with the phenotype of interest based at least in part on a meta-analysis of the one or more sets of SNPs; and
determining the combined set of SNPs based on an inverse weighting of the one or more sets of SNPs or scores from other polygenic models for at least some of the plurality of individuals.

3. The method of any one of claims 1-2, further comprising determining the plurality of individuals by filtering a dataset of individuals based on the user-selected parameters, wherein the user-selected parameters include one or more of: research consent status, missing SNP values, relatedness to other individuals in the dataset of individuals, minimum age, maximum age, sequencing platform, sex, and population classifier label.

4. The method of any one of claims 1-3, further comprising:
receiving phenotype data for the plurality of individuals; and
additionally, analyzing the phenotype data of the plurality of individuals to determine a plurality of SNPs that are statistically associated with a phenotype of interest, wherein the phenotype data includes one or more of: answers to survey questions, family history, medical records, biomarkers, and data from one or more wearable sensors.

5. The method of any one of claims 1-4, wherein the genetic data includes imputed data with greater than about 50,000,000 variants per individual, greater than about 75,000,000 variants per individual, or greater than about 100,000,000 variants per individual, or wherein a database storing the genetic data includes genetic data for greater than 10,000,000 individuals.

6. The method of any one of claims 1-5, further comprising dividing one or more of the population-specific datasets into a training set, validation set, and test set, wherein analyzing one or more of the population-specific datasets comprises analysing the genetic data of the plurality of individuals in the training set.

7. The method of claim 6, further comprising:
determining that a number of individuals in a first population-specific dataset does not exceed a first threshold;
identifying the first population-specific dataset as a test set;
determining that a number of individuals in a second population-specific dataset does not exceed a second threshold; and
dividing the second population-specific dataset into a training set and validation set.

8. The method of any one of claims 1-7, wherein the SNP filtering criteria includes one or more of:
allow listing, distance pruning, p-value threshold, and linkage disequilibrium pruning.

9. The method of any one of claims 1-8, wherein the cache of the computer system comprises genetic and phenotypic information for at least about 1,000,000 individuals, at least about 500,000 individuals, or at least about 100,000 individuals, or wherein the population-specific models include more than about 3,000 SNPs, more than about 5,000 SNPs, more than about 10,000 SNPs, more than about 50,000 SNPs, more than about 100,000 SNPs, or more than about 200,000 SNPs.

10. The method of any one of claims 1-9, wherein the plurality of population-specific models comprise a model for one or more ethnicities selected from the group consisting of: European, African American, Sub-Saharan African, North Africa, LatinX, Central America, East Asian, South Asian, Southeast Asian, West Asian, and Central Asian.

11. The method of any one of claims 1-10, further comprising: deleting the genetic and phenotypic information for the plurality of individuals in each population-specific genetic dataset within 30 days of loading the genetic and phenotypic information for the plurality of individuals in each genetic dataset into the cache.

12. The method of any one of claims 1-11, wherein the user-selected parameters comprise one or more parameters from the group consisting of: the phenotype of interest, SNPs previously determined to be associated with the phenotype of interest, prior GWAS results for the phenotype of interest, thresholds for dividing the population-specific genetic datasets into training, validation, and test sets, imputation panels, GWAS covariates including sex, age, sequencing platform, and/or principal components, lower limit for SNPs to be included in SNP sets, upper limit for SNPs to be included in SNP sets, a plurality of thresholds for p-values used to determine SNP sets, distance between SNPs in SNP sets, allow list for SNPs, disallow list for SNPs, phenotypic feature to include in model training, type of model to train, hyperparameters for training models, one or more performance metrics for evaluating models, and population-specific ethnicities for which to train a PRS model.

13. The method of any one of claims 1-12, further comprising:
storing metadata associated with one or more of the population-specific models, wherein the metadata comprises one or more of: number of SNPs; SNP selection parameters; area under the curve (AUC) values of the population-specific model; AUC values of a promoted model based on the genetic data and one or more metrics from the group consisting of: age, sex, sequencing platform, and population classifier label; R-squared; relative risk (top vs. bottom and top vs. middle); observed absolute risk difference for the phenotype (top vs. bottom, top vs. middle); or model specification.

14. The method of any one of claims 1-13, further comprising:
providing a user's data to one of the population-specific models, based on the user's ancestry, to generate a PRS score; and
generating a user report on the phenotype of interest based on the PRS score, wherein the user report comprises outcomes for the phenotype of interest including "Increased Likelihood", "Typical Likelihood", "Not Determined", "Not Applicable".

15. A non-transient computer-readable medium comprising program instructions for causing a computer to generate a polygenic risk score (PRS) model to predict phenotypes of a user, the program instructions comprising the operations of any of one claims 1-14.

## Patentansprüche

1. Verfahren zum Erzeugen eines polygenen Risiko-Score(PRS)-Modells zum Vorhersagen von Phänotypen eines Benutzers, wobei das Verfahren Folgendes umfasst:
Empfangen von benutzerausgewählten Parametern in Bezug auf ein PRS-Modell, beinhaltend einen Phänotyp von Interesse;
Auswählen und Erhalten genetischer Daten für eine Vielzahl von Individuen basierend auf den benutzerausgewählten Parametern, wobei die genetischen Daten Daten beinhalten, die sich auf ein Vorhandensein oder Fehlen des Phänotyps von Interesse in der Vielzahl von Individuen beziehen;
Bestimmen einer Vielzahl populationsspezifischer genetischer Datensätze aus den genetischen Daten basierend auf der Vielzahl von Individuen;
Analysieren der populationsspezifischen genetischen Datensätze, um einen oder mehrere Sätze von SNPs zu bestimmen, die statistisch mit dem Phänotyp von Interesse für diesen populationsspezifischen genetischen Datensatz assoziiert sind, wobei jeder Satz von SNPs einem populationsspezifischen genetischen Datensatz entspricht;
Anwenden von SNP-Filterkriterien auf den einen oder die mehreren Sätze von SNPs, um eine Vielzahl von Trainings-SNP-Sätzen zu erzeugen, wobei die SNP-Filterkriterien zumindest teilweise auf den benutzerausgewählten Parametern basieren;
Laden von genetischen und phänotypischen Informationen für die Vielzahl von Individuen in jedem populationsspezifischen genetischen Datensatz in einen Cache eines Computersystems;
Trainieren einer Vielzahl von Modellen unter Verwendung von Maschinenlerntechniken basierend zumindest teilweise auf den genetischen und phänotypischen Informationen für die Vielzahl von Individuen in dem Cache, der Vielzahl von Trainings-SNP-Sätzen und dem Phänotyp von Interesse;
Erzeugen einer oder mehrerer Leistungsmetriken für jedes der Vielzahl von Modellen für jeden der populationsspezifischen genetischen Datensätze; und
Bestimmen einer Vielzahl von populationsspezifischen Modellen basierend auf der einen oder den mehreren Leistungsmetriken.

2. Verfahren nach Anspruch 1, ferner umfassend:
Bestimmen eines kombinierten Satzes von SNPs, die statistisch mit dem Phänotyp von Interesse assoziiert sind, basierend zumindest teilweise auf einer Meta-Analyse des einen oder der mehreren Sätze von SNPs; und
Bestimmen des kombinierten Satzes von SNPs basierend auf einer inversen Gewichtung des einen oder der mehreren Sätze von SNPs oder Punktwertungen aus anderen polygenen Modellen für mindestens einige der Vielzahl von Individuen.

3. Verfahren nach einem der Ansprüche 1-2, ferner umfassend Bestimmen der Vielzahl von Individuen durch Filtern eines Datensatzes von Individuen basierend auf den benutzerausgewählten Parametern, wobei die benutzerausgewählten Parameter eines oder mehrere von Folgendem beinhalten: Forschungseinwilligungsstatus, fehlende SNP-Werte, Verwandtschaft mit anderen Individuen in dem Datensatz von Individuen, minimales Alter, maximales Alter, Sequenzierplattform, Geschlecht und Populationsklassifizierer-Label.

4. Verfahren nach einem der Ansprüche 1-3, ferner umfassend:
Empfangen von Phänotypdaten für die Vielzahl von Individuen; und
zusätzlich dazu Analysieren der Phänotypdaten der Vielzahl von Individuen, um eine Vielzahl von SNPs zu bestimmen, die mit einem Phänotyp von Interesse statistisch assoziiert sind, wobei die Phänotypdaten eines oder mehrere von Folgendem beinhalten: Antworten auf Fragen in Fragebögen, Familiengeschichte, medizinische Aufzeichnungen, Biomarker und Daten aus einem oder mehreren tragbaren Sensoren.

5. Verfahren nach einem der Ansprüche 1-4, wobei die genetischen Daten imputierte Daten mit mehr als etwa 50.000.000 Varianten pro Individuum, mehr als etwa 75.000.000 Varianten pro Individuum oder mehr als etwa 100.000.000 Varianten pro Individuum beinhalten, oder wobei eine Datenbank, die die genetischen Daten speichert, genetische Daten für mehr als 10.000.000 Individuen beinhaltet.

6. Verfahren nach einem der Ansprüche 1-5, ferner umfassend das Aufteilen eines oder mehrerer der populationsspezifischen Datensätze in einen Trainingssatz, einen Validierungssatz und einen Testsatz, wobei das Analysieren eines oder mehrerer der populationsspezifischen Datensätze das Analysieren der genetischen Daten der Vielzahl von Individuen im Trainingssatz umfasst.

7. Verfahren nach Anspruch 6, ferner umfassend:
Feststellen, dass eine Anzahl von Individuen in einem ersten populationsspezifischen Datensatz einen ersten Schwellenwert nicht überschreitet;
Identifizieren des ersten populationsspezifischen Datensatzes als einen Testsatz;
Bestimmen, dass eine Anzahl von Individuen in einem zweiten populationsspezifischen Datensatz einen zweiten Schwellenwert nicht überschreitet; und
Aufteilen des zweiten populationsspezifischen Datensatzes in einen Trainingssatz und einen Validierungssatz.

8. Verfahren nach einem der Ansprüche 1-7, wobei das SNP-Filterkriterium eines oder mehrere von Folgendem beinhaltet:
Zulässigkeits-Liste, Distanzschnitt, p-Wert-Schwelle und Kopplungsungleichgewicht-Schnitt.

9. Verfahren nach einem der Ansprüche 1-8, wobei der Cache des Computersystems genetische und phänotypische Informationen für mindestens etwa 1.000.000 Individuen, mindestens etwa 500.000 Individuen oder mindestens etwa 100.000 Individuen umfasst, oder wobei die populationsspezifischen Modelle mehr als etwa 3.000 SNPs, mehr als etwa 5.000 SNPs, mehr als etwa 10.000 SNPs, mehr als etwa 50.000 SNPs, mehr als etwa 100.000 SNPs oder mehr als etwa 200.000 SNPs beinhalten.

10. Verfahren nach einem der Ansprüche 1-9, wobei die Vielzahl von populationsspezifischen Modellen ein Modell für eine oder mehrere Ethnizitäten umfassen, ausgewählt aus der Gruppe bestehend aus: Europäisch, Afroamerikanisch, Afrikanisch-südlich der Sahara, Nordafrika, LatinX, Mittelamerika, Ostasiatisch, Südasiatisch, Südostasiatisch, Westasiatisch und Zentralasiatisch.

11. Verfahren nach einem der Ansprüche 1-10, ferner umfassend:
Löschen der genetischen und phänotypischen Informationen für die Vielzahl von Individuen in jedem populationsspezifischen genetischen Datensatz innerhalb von 30 Tagen nach dem Laden der genetischen und phänotypischen Informationen für die Vielzahl von Individuen in jedem genetischen Datensatz in den Cache.

12. Verfahren nach einem der Ansprüche 1-11, wobei die benutzerausgewählten Parameter einen oder mehrere Parameter aus der Gruppe umfassen, die aus Folgendem besteht: dem Phänotyp von Interesse, SNPs, die zuvor als mit dem Phänotyp von Interesse assoziiert bestimmt wurden, früheren GWAS-Ergebnissen für den Phänotyp von Interesse, Schwellenwerten zum Aufteilen der populationsspezifischen genetischen Datensätze in Trainings-, Validierungs- und Testsätze, Imputations-Panels, GWAS-Kovariablen einschließlich Geschlecht, Alter, Sequenzierplattform und/oder Hauptkomponenten, Untergrenze für SNPs, die in SNP-Sätzen eingeschlossen sein sollen, Obergrenze für SNPs, die in SNP-Sätzen eingeschlossen sein sollen, einer Vielzahl von Schwellenwerten für p-Werte, die zum Bestimmen von SNP-Sätzen verwendet werden, Distanz zwischen SNPs in SNP-Sätzen, Zulässigkeits-Liste für SNPs, Nichtzulässigkeits-Liste für SNPs, phänotypischem Merkmal zum Einschluss im Modelltraining, Typ des zu trainierenden Modells, Hyperparametern für Trainingsmodelle, einer oder mehreren Leistungsmetriken zum Evaluieren von Modellen, und populationsspezifischen Ethnizitäten, für die ein PRS-Modell trainiert werden soll.

13. Verfahren nach einem der Ansprüche 1-12, ferner umfassend:
Speichern von Metadaten, die mit einem oder mehreren der populationsspezifischen Modelle assoziiert sind, wobei die Metadaten eines oder mehrere der Folgenden umfassen: Anzahl von SNPs; SNP-Auswahlparameter; Fläche-unter-der-Kurve (AUC)-Werte des populationsspezifischen Modells; AUC-Werte eines geförderten Modells basierend auf den genetischen Daten, und einer oder mehreren Metriken aus der Gruppe bestehend aus: Alter, Geschlecht, Sequenzierplattform und Populationsklassifizierer-Label; R-Quadrat; relatives Risiko (hoch vs. klein, hoch vs. mittel); beobachteter Unterschied des absoluten Risikos für den Phänotyp (hoch vs. klein, hoch vs. mittel) oder Modellspezifikation.

14. Verfahren nach einem der Ansprüche 1-13, ferner umfassend:
Bereitstellen der Daten eines Benutzers an eines der populationsspezifischen Modelle basierend auf den Vorfahren des Benutzers, um eine PRS-Bewertung zu erzeugen; und
Erstellen eines Benutzerberichts über den Phänotyp von Interesse basierend auf dem PRS-Score, wobei der Benutzerbericht Ergebnisse für den Phänotyp von Interesse umfasst, einschließlich "Erhöhte Wahrscheinlichkeit", "Typische Wahrscheinlichkeit", "Nicht bestimmt", "Nicht zutreffend".

15. Nichtflüchtiges computerlesbares Medium, das Programmanweisungen umfasst, um einen Computer zu veranlassen, ein polygenes-Risiko-Score(PRS)-Modell zu erzeugen, um Phänotypen eines Benutzers vorherzusagen, wobei die Programmanweisungen die Betriebsvorgänge nach einem der Ansprüche 1-14 umfassen.

## Revendications

1. Procédé de génération d'un modèle de score de risque polygénique (PRS) pour prédire des phénotypes d'un utilisateur, le procédé comprenant :
la réception de paramètres sélectionnés par l'utilisateur liés à un modèle PRS comprenant un phénotype d'intérêt ;
la sélection et l'obtention de données génétiques pour une pluralité d'individus sur la base des paramètres sélectionnés par l'utilisateur, les données génétiques comprenant des données concernant la présence ou l'absence du phénotype d'intérêt chez la pluralité d'individus ;
la détermination d'une pluralité d'ensembles de données génétiques spécifiques à une population à partir des données génétiques basées sur la pluralité d'individus ;
l'analyse des ensembles de données génétiques spécifiques à une population pour déterminer un ou plusieurs ensembles de polymorphismes mononucléotidiques (SNP)qui sont associés statistiquement au phénotype d'intérêt pour cet ensemble de données génétiques spécifique à une population, chaque ensemble de SNP correspondant à un ensemble de données génétiques spécifique à une population ;
l'application de critères de filtrage de SNP aux un ou plusieurs ensembles de SNP pour générer une pluralité d'ensembles de SNP d'entraînement, les critères de filtrage de SNP étant basés, au moins en partie, sur les paramètres sélectionnés par l'utilisateur ;
le chargement dans une mémoire cache d'un système informatique d'informations génétiques et phénotypiques pour la pluralité d'individus dans chaque ensemble de données génétiques spécifique à une population ;
l'entraînement d'une pluralité de modèles au moyen de techniques d'apprentissage automatique basées, au moins en partie, sur les informations génétiques et phénotypiques pour la pluralité d'individus dans la mémoire cache, la pluralité d'ensembles de SNP d'entraînement et le phénotype d'intérêt ;
la génération d'une ou plusieurs mesures de performance pour chaque modèle de la pluralité de modèles pour chacun des ensembles de données génétiques spécifiques à une population ; et
la détermination d'une pluralité de modèles spécifiques à une population sur la base de la ou des mesures de performance.

2. Procédé selon la revendication 1, comprenant en outre :
la détermination d'un ensemble combiné de SNP qui sont associés statistiquement au phénotype d'intérêt sur la base, au moins en partie, d'une métaanalyse du ou des ensembles de SNP ; et
la détermination de l'ensemble combiné de SNP sur la base d'une pondération inverse du ou des ensembles de SNP ou scores d'autres modèles polygéniques pour au moins certains individus de la pluralité d'individus.

3. Procédé selon l'une quelconque des revendications 1 à 2, comprenant en outre la détermination de la pluralité d'individus par filtrage d'un ensemble de données d'individus sur la base des paramètres sélectionnés par l'utilisateur, dans lequel les paramètres sélectionnés par l'utilisateur comprennent un ou plusieurs des éléments suivants : le statut de consentement de recherche, les valeurs de SNP manquantes, le lien de parenté avec d'autres individus dans l'ensemble de données d'individus, l'âge minimal, l'âge maximal, la plateforme de séquençage, le sexe et l'étiquette de classification de population.

4. Procédé selon l'une quelconque des revendications 1 à 3, comprenant en outre :
la réception de données phénotypiques pour la pluralité d'individus ; et
en outre, l'analyse des données phénotypiques de la pluralité d'individus pour déterminer une pluralité de SNP qui sont associés statistiquement à un phénotype d'intérêt, les données phénotypiques comprenant un ou plusieurs des éléments suivants : des réponses à questions d'enquête, des antécédents familiaux, des dossiers médicaux, des biomarqueurs et des données provenant d'un ou plusieurs capteurs à porter sur soi.

5. Procédé selon l'une quelconque des revendications 1 à 4, dans lequel les données génétiques comprennent des données imputées avec plus d'environ 50 000 000 variants par individu, plus d'environ 75 000 000 variants par individu, ou plus d'environ 100 000 000 variants par individu, ou dans lequel une base de données stockant les données génétiques comprend des données génétiques pour plus de 10 000 000 individus.

6. Procédé selon l'une quelconque des revendications 1 à 5, comprenant en outre la division d'un ou plusieurs des ensembles de données spécifiques à une population en un ensemble d'entraînement, un ensemble de validation et un ensemble de test, dans lequel l'analyse d'un ou plusieurs des ensembles de données spécifiques à une population comprend l'analyse des données génétiques de la pluralité d'individus dans l'ensemble d'entraînement.

7. Procédé selon la revendication 6, comprenant en outre :
la détermination du fait qu'un nombre d'individus dans un premier ensemble de données spécifique à une population ne dépasse pas un premier seuil ;
l'identification du premier ensemble de données spécifique à une population en tant qu'ensemble de test ;
la détermination du fait qu'un nombre d'individus dans un deuxième ensemble de données spécifique à une population ne dépasse pas un deuxième seuil ; et
la division du deuxième ensemble de données spécifique à une population en un ensemble d'entraînement et un ensemble de validation.

8. Procédé selon l'une quelconque des revendications 1 à 7, dans lequel les critères de filtrage de SNP comprennent l'un ou plusieurs parmi :
l'inscription sur une liste d'autorisation, l'élagage en fonction de la distance, le seuil de valeur p et l'élagage en fonction du déséquilibre de liaison

9. Procédé selon l'une quelconque des revendications 1 à 8, dans lequel la mémoire cache du système informatique comprend des informations génétiques et phénotypiques pour au moins environ 1 000 000 individus, au moins environ 500 000 individus, ou au moins environ 100 000 individus, ou dans lequel les modèles spécifiques à une population comprennent plus d'environ 3 000 SNP, plus d'environ 5 000 SNP, plus d'environ 10 000 SNP, plus d'environ 50 000 SNP, plus d'environ 100 000 SNP ou plus d'environ 200 000 SNP.

10. Procédé selon l'une quelconque des revendications 1 à 9, dans lequel la pluralité de modèles spécifiques d'une population comprend un modèle pour une ou plusieurs origines ethniques choisies dans le groupe constitué par : européen, afro-américain, Afrique subsaharienne, Afrique du Nord, latinx, Amérique centrale, Asie de l'Est, Asie du Sud, Asie du Sud-Est, Asie de l'Ouest et Asie centrale.

11. Procédé selon l'une quelconque des revendications 1 à 10, comprenant en outre :
la suppression des informations génétiques et phénotypiques pour la pluralité d'individus dans chaque ensemble de données génétiques spécifique à une population dans les 30 jours suivant le chargement des informations génétiques et phénotypiques pour la pluralité d'individus dans chaque ensemble de données génétiques dans la mémoire cache.

12. Procédé selon l'une quelconque des revendications 1 à 11, dans lequel les paramètres sélectionnés par l'utilisateur comprennent un ou plusieurs paramètres du groupe constitué par : le phénotype d'intérêt, les SNP précédemment déterminés comme étant associés au phénotype d'intérêt, les résultats GWAS antérieurs pour le phénotype d'intérêt, les seuils pour la division des ensembles de données génétiques spécifiques à une population en ensembles d'entraînement, de validation et de test, les panels d'imputation, les covariables GWAS comprenant le sexe, l'âge, la plateforme de séquençage et/ou les composantes principales, la limite inférieure pour les SNP à inclure dans les ensembles de SNP, la limite supérieure pour les SNP à inclure dans les ensembles de SNP, une pluralité de seuils pour les valeurs p utilisées pour déterminer les ensembles de SNP, la distance entre les SNP dans les ensembles de SNP, l'inscription sur une liste d'autorisation pour les SNP, l'inscription sur une liste d'interdiction pour les SNP, une caractéristique phénotypique à inclure dans l'entraînement de modèles, des hyperparamètres pour l'entraînement de modèles, une ou plusieurs mesures de performance pour évaluer les modèles, et les origines ethniques spécifiques à une population pour lesquelles un modèle PRS doit être entraîné.

13. Procédé selon l'une quelconque des revendications 1 à 12, comprenant en outre :
le stockage de métadonnées associées à un ou plusieurs des modèles spécifiques à une population, les métadonnées comprenant un ou plusieurs des éléments suivants : le nombre de SNP ; les paramètres de sélection de SNP ; les valeurs d'aire sous la courbe (ASC) du modèle spécifique à une population ; les valeurs d'ASC d'un modèle promu sur la base des données génétiques et d'une ou plusieurs mesures du groupe constitué par : l'âge, le sexe, la plateforme de séquençage et l'étiquette de classification de population ; le R-carré ; le risque relatif (haut vs. Bas et haut vs. intermédiaire) ; la différence de risque absolu observée pour le phénotype (haut vs. bas, haut vs. intermédiaire) ; ou la spécification du modèle.

14. Procédé selon l'une quelconque des revendications 1 à 13, comprenant en outre :
l'introduction des données d'un utilisateur dans l'un des modèles spécifiques à une population, sur la base de l'ascendance de l'utilisateur, pour générer un score PRS ; et
la génération d'un rapport utilisateur sur le phénotype d'intérêt sur la base du score PRS, le rapport utilisateur comprenant des résultats pour le phénotype d'intérêt, comprenant « probabilité accrue », « probabilité typique », « non déterminé », « non applicable ».

15. Support lisible par ordinateur non transitoire comprenant des instructions de programme pour amener un ordinateur à générer un modèle de score de risque polygénique (PRS) pour prédire des phénotypes d'un utilisateur, les instructions de programme comprenant les opérations selon l'une quelconque des revendications 1 à 14.
